(19) Europäisches Patentamt
European Patent Office
Office européen des brevets

(11) EP 0 599 688 B1

# (12) FASCICULE DE BREVET EUROPEEN

(45) Date de publication et mention de la délivrance du brevet:
**27.08.1997 Bulletin 1997/35**

(51) Int Cl.[6]: **C07C 41/09**, C07B 41/04, C07C 43/205

(21) Numéro de dépôt: **93402762.4**

(22) Date de dépôt: **15.11.1993**

(54) **Procédé de O-alkylation des composés phénoliques**

Verfahren zur O-Alkylierung von phenolischen Verbindungen

Process for O-alkylation of phenolic compounds

(84) Etats contractants désignés:
**DE FR GB IT NL SE**

(30) Priorité: **25.11.1992 FR 9214154**

(43) Date de publication de la demande:
**01.06.1994 Bulletin 1994/22**

(73) Titulaire: **RHONE-POULENC CHIMIE**
**92408 Courbevoie Cédex (FR)**

(72) Inventeurs:
- **Gilbert, Laurent**
  **F-69007 Lyon (FR)**
- **Janin, Marcelle**
  **F-69006 Lyon (FR)**
- **Le Govic, Anne-Marie**
  **F-75010 Paris (FR)**
- **Tirel, Philippe-Jean**
  **F-69360 Communay (FR)**

(74) Mandataire: **Dutruc-Rosset, Marie-Claude et al**
**RHONE-POULENC CHIMIE,**
**Direction de la Propriété Industrielle,**
**25, Quai Paul Doumer**
**92408 Courbevoie Cédex (FR)**

(56) Documents cités:
**EP-A- 0 420 756** **EP-A- 0 440 555**
**US-A- 4 450 306** **US-A- 4 675 456**

- **Duval, C. et al., "Dictionnaire de la chimie et de ses applications", Paris 1959, p. 1198.**
- **Kirk-Othmer "Encyclopedia of chemical technology", 3rd edition, volume 19, p. 833.**
- **Pascal, P., Ed., "Nouveau traité de chimie minérale", Tome VII, Paris 1959, p. 5.**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 599 688 B1

## Description

La présente invention a pour objet un procédé de O-alkylation des composés phénoliques. L'invention concerne plus particulièrement un procédé de préparation des éthers dérivés de diphénols.

Il existe de très nombreux procédés d'obtention d'éthers dérivés de composés phénoliques.

Un des procédés classiques pour les préparer consiste à faire réagir le phénol et l'alcool, en phase liquide ou en phase gazeuse, en présence d'un catalyseur.

Ainsi, on a décrit dans FR-A-2 149 461, un procédé de préparation d'éthers monométhyliques de diphénols, en phase liquide, qui consiste à faire réagir à une température de 200°C à 240°C, le diphénol à éthérifier, avec du méthanol, en présence de phosphate de bore comme catalyseur. Ce procédé nécessite de travailler sous forte pression, donc de faire appel à un appareillage particulier.

Par ailleurs, de nombreux documents mentionnent la préparation d'éthers de diphénols, en phase gazeuse.

Il est connu selon DE-A-827 803, de préparer l'éther monométhylique de la pyrocatéchine, en faisant réagir en phase gazeuse, entre 250°C et 300°C, la pyrocatéchine et le méthanol, en présence de phosphate de bore ou de vermicelle de silicagel imprégné d'acide phosphorique. Le taux de conversion obtenu de la pyrocatéchine est de 64,9 %, le degré de sélectivité vis-à-vis du gaïacol est de 54,6 % et de 11,5 % par rapport au vératrole.

Dans le cas où l'on utilise le phosphate de bore comme catalyseur, ce catalyseur possède une bonne activité catalytique puisque le taux de conversion de la pyrocatéchine est de 50 à 65 % et une bonne sélectivité en gaïacol d'environ 80 à 90 %. Toutefois, lorsque ce catalyseur est mis en oeuvre dans un procédé en continu, ce catalyseur ne donne plus satisfaction car le phosphate de bore réagit avec la pyrocatéchine et se trouve élué par la pyrocatéchine. Il s'ensuit une perte catalytique et donc une baisse de l'activité catalytique lors d'une opération de longue durée.

Pour pallier cet inconvénient, on a proposé de mettre en oeuvre un catalyseur contenant de l'aluminium, du bore et du phosphore (FR-A-2 303 784). Un tel catalyseur permet une bonne conversion du diphénol mais bien que sa durée de vie soit supérieure, celle-ci doit être améliorée car il y a également consommation du phosphate de bore.

On a proposé, selon EP-A-0 420 756, pour résoudre ce problème technique, de faire appel à un catalyseur constitué d'une phase active comprenant un composé du phosphore tel que l'acide phosphorique et/ou un produit de réaction d'un composé du phosphore et d'un composé du bore tel que le phosphate de bore : ladite phase active étant déposée sur un support. Afin de compenser la perte catalytique, il est préconisé d'introduire en continu la phase active avec les gaz d'alimentation, ce qui permet l'imprégnation du support par la phase active. Il en résulte des problèmes de purification du produit obtenu qui contient du borate de méthyle et de l'acide phosphorique.

On a décrit comme catalyseurs d'alkylation des phénols, dans US 4 675 456, un catalyseur comprenant un oxysulfate de lanthanide et dans US 4 450 306, $La_2(HPO_4)_3$, $Sr(HSO_4)_3$ et $Ba(HSO_4)_3$, utilisés en phase gazeuse ou liquide.

Un des objectifs de la présente invention est de fournir un procédé de O-alkylation des phénols mono- ou polyhydroxylés qui permette d'éviter les inconvénients précités et apporte une autre solution au problème de la perte d'activité catalytique.

Un autre objectif de la présente invention est de disposer d'un catalyseur qui permet de préparer des diphénols O-alkylés tout en modulant la production des éthers phénoliques obtenus. En effet, selon la demande du marché, il peut être intéressant de disposer d'un procédé qui, selon le catalyseur choisi, permette d'obtenir sélectivement un éther de monoalcoyle de diphénol ou bien, un mélange d'un éther de monoalcoyle de diphénol et d'un éther de dialcoyle de diphénol.

La présente invention permet d'atteindre ces objectifs.

Il a maintenant été trouvé et c'est ce qui constitue l'objet de la présente invention, un procédé de O-alkylation d'un composé phénolique caractérisé par le fait qu'il consiste à faire réagir ledit composé phénolique avec un alcanol, en phase gazeuse, en présence d'une quantité efficace d'un catalyseur choisi parmi les orthophosphates de métaux de terres rares trivalentes.

Dans l'exposé qui suit de la présente invention, on entend par "composé phénolique", un composé aromatique porteur d'au moins un groupe hydroxyle. On entend par "composé aromatique", la notion classique d'aromaticité telle que définie dans la littérature, notamment par Jerry MARCH, Advanced Organic Chemistry, 3ème édition, John Wiley and Sons, 1985, p. 37 et suivantes.

Par "métaux de terres rares", on entend les lanthanides ayant un numéro atomique de 57 à 71 et l'yttrium ainsi que le scandium.

Par "orthophosphates de métaux de terres rares trivalentes", on entend un composé dans lequel le rapport molaire entre l'anion phosphate et le cation terre rare trivalente est de $1 \pm 0,2$, de préférence égal à 1,0.

Une première variante du procédé de l'invention consiste à faire appel à un orthophosphate de terre rare calciné à haute température.

Une autre variante de l'invention qui est préférée, consiste à faire appel à un orthophosphate de terre rare trivalente imprégné par un métal alcalin ou un métal alcalino-terreux.

Dans le présente texte, on appelle "agent dopant", l'élément alcalin ou alcalino-terreux.

Conformément au procédé de l'invention, on fait réagir un composé phénolique avec un alcanol, en phase gazeuse, en présence d'une quantité efficace d'un catalyseur choisi parmi les phosphates des métaux de terres rares dopés ou non.

Plus précisément, la présente invention a pour objet un procédé de O-alkylation d'un composé phénolique de formule générale (I) :

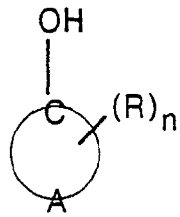

(I)

dans laquelle :

- A symbolise un reste d'un radical carbocyclique aromatique, monocyclique ou polycyclique ou un radical divalent constitué par un enchaînement de deux ou plusieurs radicaux carbocycliques aromatiques monocycliques,
- R représente un ou plusieurs substituants, identiques ou différents,
- n est un nombre inférieur ou égal à 5.

avec un alcanol répondant à la formule générale (II) :

$$R'\text{-}OH \qquad (II)$$

dans ladite formule (II) :

- R' représente un radical hydrocarboné ayant de 1 à 24 atomes de carbone, qui peut être un radical aliphatique acyclique saturé ou insaturé, linéaire ou ramifié ; un radical cycloaliphatique saturé ou insaturé, monocyclique ou polycyclique ; un radical aliphatique saturé ou insaturé, linéaire ou ramifié, porteur d'un substituant cyclique.

On utilisera ci-après le terme "alcanol" de manière générique pour désigner tous les alcanols répondant à la formule (II).

Conformément au procédé de l'invention, on fait réagir le composé phénolique de formule (I) et l'alcanol, en phase gazeuse. On entend par cette expression que les différents réactifs sont vaporisés dans les conditions réactionnelles mais le procédé n'exclut pas la présence d'une éventuelle phase liquide résultant soit des propriétés physiques des réactifs, soit d'une mise en oeuvre sous pression ou de l'utilisation d'un solvant organique.

Le procédé de l'invention s'applique à tout composé phénolique répondant à la formule générale (I) et, plus particulièrement, aux composés phénoliques de formule (I) dans laquelle :

- le ou les radicaux R représentent l'un des groupes suivants :
  - . un atome d'hydrogène,
  - . un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle,
  - . un radical alcényle linéaire ou ramifié ayant de 2 à 6 atomes de carbone, de préférence, de 2 à 4 atomes de carbone, tel que vinyle, allyle,
  - . un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy,
  - . un groupe acyle ayant de 2 à 6 atomes de carbone,
  - . un radical de formule :

$$-R_1\text{-}OH$$

$-R_1-COOR_2$

$-R_1-CHO$

$-R_1-NO_2$

$-R_1-CN$

$-R_1-(NR_2)_2$

$-R_1-CO-(NR_2)_2$

$-R_1-X$

$-R_1-CF_3$

dans lesdites formules, $R_1$ représente un lien valentiel ou un radical hydrocarboné divalent, linéaire ou ramifié, saturé ou insaturé, ayant de 1 à 6 atomes de carbone tel que, par exemple, méthylène, éthylène, propylène, isopropylène, isopropylidène ; $R_2$ représente un atome d'hydrogène ou un radical alkyle linéaire ou ramifié ayant de 1 à 6 atomes de carbone ; X symbolise un atome d'halogène, de préférence un atome de chlore, de brome ou de fluor.

Le composé phénolique de formule (I) peut être porteur d'un ou plusieurs substituants. Des exemples de substituants sont donnés ci-dessus mais cette liste ne présente pas de caractère limitatif. N'importe quel substituant peut être présent sur le cycle dans la mesure où il n'interfère pas au niveau du produit désiré. Si le substituant est porteur d'un atome d'hydrogène, il est possible qu'il soit remplacé par le radical R' apporté par l'alcanol ou s'il est porteur d'un groupe alkyle $R_2$ celui-ci sera échangé par R' si R' et $R_2$ sont des groupes alkyle de nature différente.

La présente invention s'applique tout particulièrement aux composés phénoliques répondant à la formule (I) dans laquelle R représente :

. un atome d'hydrogène
. un groupe OH,
. un radical alkyle linéaire ou ramifié ayant de 1 à 6 atomes de carbone,
. un radical alcényle linéaire ou ramifié ayant de 2 à 6 atomes de carbone,
. un radical alkoxy linéaire ou ramifié ayant de 1 à 6 atomes de carbone,
. un groupe -CHO,
. un groupe acyle ayant de 2 à 6 atomes de carbone,
. un groupe $-COOR_2$ où $R_2$ a la signification donnée précédemment,
. un groupe $-NO_2$,
. un atome d'halogène, de préférence, de fluor, de chlore, de brome,
. un groupe $-CF_3$.

- n est un nombre égal à 0, 1, 2 ou 3.

Parmi les composés de formule (I), on met en oeuvre plus particulièrement ceux dont le reste (A) représente :

- un radical carbocyclique aromatique monocyclique ou polycyclique avec des cycles pouvant former entre eux un système orthocondensé répondant à la formule (Ia) :

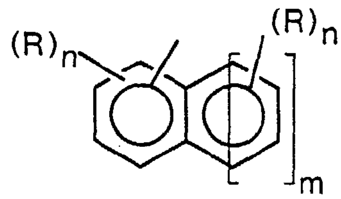

(Ia)

dans ladite formule (Ia), m représente un nombre égal à 0, 1 ou 2 et les symboles R et n identiques ou différents ayant la signification donnée précédemment,

- un radical constitué par un enchaînement de deux ou plusieurs radicaux carbocycliques aromatiques monocycliques répondant à la formule (Ib) :

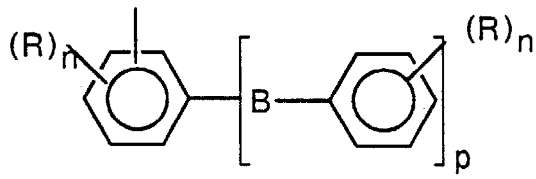

(Ib)

dans ladite formule (Ib), les symboles R et n identiques ou différents ont la signification donnée précédemment, p est un nombre égal à 0, 1, 2 ou 3 et B représente:

- un lien valentiel
- un radical alkylène ou alkylidène ayant de 1 à 4 atomes de carbone, de préférence, un radical méthylène ou isopropylidène,
- un des groupes suivants :
  -O-, -CO-, -COO-, -OCOO-
  -S-, -SO-, $-SO_2-$,

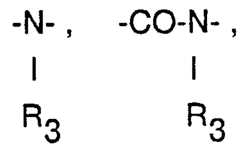

dans ces formules, $R_3$ représente un atome d'hydrogène ou un radical alkyle ayant de 1 à 4 atomes de carbone, un radical cyclohexyle ou phényle.

Les composés de formule (I) mis en oeuvre préférentiellement répondent aux formules (Ia) et (Ib) dans lesquelles :

- R représente un atome d'hydrogène, un groupe hydroxyle, un groupe _CHO, un groupe $-NO_2$, un radical alkyle ou alkoxy linéaire ou ramifié ayant de 1 à 6 atomes de carbone, de préférence, de 1 à 4 atomes de carbone,
- B symbolise un lien valentiel, un radical alkylène ou alkylidène ayant de 1 à 4 atomes de carbone ou un atome d'oxygène,
- m est égal à 0 ou 1,
- n est égal à 0, 1 ou 2,
- p est égal à 0 ou 1.

Encore plus préférentiellement, on choisit les composés de formule (I) dans laquelle R représente un atome d'hydrogène, un groupe hydroxyle, un radical méthyle, un radical méthoxy.

A titre illustratif de composés répondant à la formule (I), on peut mentionner plus particulièrement :

- ceux dans lesquels le reste A répond à la formule (Ia) dans laquelle m et n sont égaux à 0, tels que le phénol,
- ceux dans lesquels le reste A répond à la formule (Ia) dans laquelle m est égal à 0 et n est égal à 1, tels que :

- . l'hydroquinone
- . la pyrocatéchine
- . la résorcine
- . l'o-crésol
- . le m-crésol
- . le p-crésol
- . l'éthyl-2 phénol
- . l'éthyl-3 phénol
- . l'éthyl-4 phénol
- . le propyl-2 phénol
- . le propyl-4 phénol
- . le sec-butyl-2 phénol
- . le tert-butyl-2 phénol
- . le tert-butyl-3 phénol
- . le sec-butyl-4 phénol
- . le tert-butyl-4 phénol
- . le méthoxy-2 phénol
- . le méthoxy-3 phénol
- . le méthoxy-4 phénol
- . l'éthoxy-2-phénol
- . l'éthoxy-3-phénol
- . l'éthoxy-4 phénol
- . l'aldéhyde salicylique
- . le p-hydroxybenzaldéhyde
- . le salicylate de méthyle
- . l'ester méthylique de l'acide p-hydroxybenzoique
- . le chloro-2 phénol
- . le chloro-3 phénol
- . le chloro-4 phénol
- . le nitro-2 phénol
- . le nitro-3 phénol
- . le nitro-4 phénol
- . le N-acétyl p-aminophénol

- ceux dans lesquels le reste A répond à la formule (Ia) dans laquelle m est égal à O et n est égal à 2, tels que :

- . le diméthyl-2,3 phénol
- . le diméthyl-2,4 phénol
- . le diméthyl-2,5 phénol
- . le diméthyl-2,6 phénol
- . le diméthyl-3,4 phénol
- . le diméthyl-3,5 phénol
- . la vanilline
- . l'isovanilline
- . l'hydroxy-2 acétamido-5 benzaldéhyde
- . l'hydroxy-2 propionamido-5 benzaldéhyde
- . l'allyloxy-4 benzaldéhyde.
- . le dichloro-2 3 phénol
- . le dichloro-2,4 phénol
- . le dichloro-2,5 phénol
- . le dichloro-2,6 phénol
- . le dichloro-3,4 phénol
- . le dichloro-3,5 phénol
- . la méthylhydroquinone
- . la chlorohydroquinone
- . le pyrogallol

- ceux dans lesquels le reste A répond à la formule (Ia) dans laquelle m est égal à 0 et n est égal à 3, tels que :

. la bromo-4 vanilline
. l'hydroxy-4 vanilline
. le triméthyl 2,3,5 phénol
. le triméthyl 2,3,6 phénol
. le triméthyl 2,4,6 phénol
. le triméthyl 3,4,5 phénol
. le tri-tert-butyl-2,4,6 phénol
. le di-tert butyl-2,4 phénol
. le di-tert butyl-2,6 phénol
. le di-tert butyl-3,5 phénol
. le di-tert-butyl-2,6 méthyl-4 phénol
. le diméthyl-2,6 tert-butyl-4 phénol
. le trinitro-2,4,6 phénol
. le dichloro-2,6 nitro-4 phénol
. le trichloro-2,4,6 phénol
. le trichloro-2,3,4 phénol
. le trichloro-2,3,5 phénol
. le trichloro-2,3,6 phénol
. les dichlorohydroquinones
. le diméthoxy-3,5 hydroxy-4 benzaldéhyde

- ceux dans lesquels le reste A répond à la formule (Ia) dans laquelle m est égal à 1 et n est supérieur ou égal à 1, tels que :

. le dihydroxy-1,2 naphtalène
. le dihydroxy-1,4 naphtalène
. le dihydroxy-1,5 naphtalène
. le dihydroxy-2,3 naphtalène
. le dihydroxy-2,6 naphtalène
. le dihydroxy-2,7 naphtalène
. le méthoxy-4 naphtol-1
. le bromo-6 naphtol-2

- ceux dans lesquels le reste A répond à la formule (Ib) dans laquelle n est supérieur ou égal à 1, tels que :

. le phénoxy-2 phénol
. le phénoxy-3 phénol
. la phénylhydroquinone
. le dihydroxy-4,4' biphényl
. l'isopropylidène diphénol-4,4' (bis phénol-A)
. le bis(hydroxy-4 phényl)méthane
. le bis(hydroxy-4 phényl)sulfone
. le bis(hydroxy-4 phényl)sulfoxyde
. le tétrabromo bis-phénol A

L'alcanol qui intervient dans le procédé de l'invention répond à la formule (II) dans laquelle R' représente un radical aliphatique acyclique, saturé ou insaturé, linéaire ou ramifié.

Plus précisément, R' représente un radical alkyle, alcényle, alcadiényle, alcynyle, linéaire ou ramifié ayant de préférence de 1 à 24 atomes de carbone.

La chaîne hydrocarbonée peut être éventuellement :

- interrompue par l'un des groupes suivants :
  -O-, -CO-, -COO-, -OCOO-, -S-, $-SO_2-$,

-N- , -CO-N- ,
| |
R4 R4

dans ces formules $R_4$ représente l'hydrogène ou un radical alkyle linéaire ou ramifié ayant de 1 à 4 atomes de carbone, de préférence, un radical méthyle ou éthyle,

- et/ou porteuse de l'un des substituants suivants :
  -OH, -OCOO-, $-COOR_4$, -CHO, $-NO_2$, -X, $-CF_3$ dans ces formules, $R_4$ ayant la signification donnée précédemment.

Le reste aliphatique acyclique, saturé ou insaturé, linéaire ou ramifié peut être éventuellement porteur d'un substituant cyclique. Par cycle, on entend un cycle carbocyclique ou hétérocyclique, saturé, insaturé ou aromatique.

Le reste aliphatique acyclique peut être relié au cycle par un lien valentiel ou par l'un des groupes suivants :
-O-, -CO-, -COO-, -OCOO-, -S-, $-SO_2-$,

-N- , -CO-N- ,
| |
$R_4$ $R_4$

dans ces formules, $R_4$ ayant la signification donnée précédemment.

Comme exemples de substituants cycliques, on peut envisager des substituants cycloaliphatiques, aromatiques ou hétérocycliques, notamment cycloaliphatiques comprenant 6 atomes de carbone dans le cycle ou benzéniques, ces substituants cycliques étant eux-mêmes éventuellement porteurs de 1, 2, 3, 4 ou 5 radicaux $R_5$, identiques ou différents, $R_5$ ayant la signification donnée précédemment pour le radical R porté par le cycle de la formule (I). Les substituants cycliques préférés sont les noyaux benzéniques.

Dans la formule générale (II) des alcanols, R' peut représenter également un radical carbocyclique saturé ou comprenant 1 ou 2 insaturations dans le cycle, ayant généralement de 3 à 7 atomes de carbone, de préférence, 6 atomes de carbone dans le cycle ledit cycle pouvant être substitué par 1 à 5 radicaux $R_5$ de préférence 1 à 3, $R_5$ ayant les significations énoncées précédemment.

Comme exemples préférés de radicaux R', on peut citer les radicaux cyclohexyle ou cyclohexène-yle, éventuellement substitués par des radicaux alkyle linéaires ou ramifiés, ayant de 1 à 4 atomes de carbone.

Le procédé est mis en oeuvre aisément avec la plupart des alcanols. Comme exemples d'alcanols, on peut citer les alcanols aliphatiques inférieurs ayant de 1 à 5 atomes de carbone, tels que par exemple, le méthanol, l'éthanol, le trifluoroéthanol, le propanol, l'alcool isopropylique, le butanol, l'alcool isobutylique, l'alcool sec-butylique, l'alcool tert-butylique, le pentanol, l'alcool isopentylique, l'alcool sec-pentylique et l'alcool tert-pentylique, l'éther monoéthylique d'éthylèneglycol, le lactate de méthyle, le lactate d'isobutyle, le D-lactate de méthyle, le D-lactate d'isobutyle ainsi que les alcools aliphatiques supérieurs ayant au moins 6 et jusqu'à environ 20 atomes de carbone, tels que par exemple, l'hexanol, l'heptanol, l'alcool isoheptylique, l'octanol, l'alcool isooctylique, l'éthyl-2 hexanol, l'alcool sec-octylique, l'alcool tert-octylique, le nonanol, l'alcool isononylique, le décanol, le dodécanol, le tétradécanol, l'octadécanol, l'hexadécanol, l'alcool oléylique, l'alcool eicosylique, l'éther monoéthylique de diéthylèneglycol. On peut mettre en oeuvre des alcools cycloaliphatiques ayant de 3 à environ 20 atomes de carbone, tel que, par exemple, le cyclopropanol, le cyclobutanol, le cyclopentanol, le cyclohexanol, le cycloheptanol, le cyclooctanol, le cyclododécanol, le tripropylcyclohexanol, le méthylcyclohexanol et le méthylcycloheptanol, ou bien un alcool aliphatique porteur d'un groupement aromatique ayant de 7 à environ 20 atomes de carbone tel que par exemple l'alcool benzylique, l'alcool phénéthylique, l'alcool phénylpropylique, l'alcool phényloctadécylique et l'alcool naphtyldécylique. Il est également possible de mettre en oeuvre des polyols notamment les polyoxyéthylèneglycols tels que, par exemple, l'éthylène glycol, le diéthylène glycol, le triéthylène glycol, le propylène glycol, le glycérol.

Parmi les alcools précités, on met en oeuvre de préférence dans le procédé de l'invention, les alcools primaires ou secondaires ayant 1 à 4 atomes de carbone.

Les alcools préférés sont le méthanol, l'éthanol, l'isopropanol ou l'éthylène glycol.

Pour ce qui est du catalyseur de l'invention, le catalyseur de l'invention répond plus précisément à la formule suivante :

$$MPO_4\ (Im)_p \qquad (III)$$

dans laquelle :

- M représente:
  - . une terre rare trivalente $M_3$,
  - . ou un mélange d'au moins une terre trivalente $M_3$ et d'au moins un élément choisi parmi les métaux alcalins $M_1$ et les métaux alcalino-terreux $M_2$ avec la relation :

$$M = \alpha M_1^{+} + \beta M_2^{++} + \gamma M_3^{3+} \text{ et } \alpha + 2\beta + 3\gamma = 3$$

- Im correspond à un composé d'imprégnation basique constitué de métal alcalino-terreux, de préférence de métal alcalin et leurs mélanges associés à un contre-anion pour assurer la neutralité électrique,
- $\alpha$ est un coefficient compris entre 0 et 3, avantageusement supérieur à 0,01 et au plus égal à 0,5, de préférence compris entre 0,05 et 0,2,
- $\beta$ est un coefficient compris entre 0 et 3/2, de préférence entre 0 et 1/3 ou bien $1 \pm 0{,}1$,
- $\gamma$ est un coefficient compris entre 0 et 1, avantageusement au moins égal à 1/3, de préférence à 1/2,
- p est inférieur à 0,5 et avantageusement compris entre 0,04 et 0,25.

Pour la définition des éléments, on se réfère ci-après à la Classification périodique des éléments publiée dans le Bulletin de la Société Chimique de France, n°1 (1966).

Dans la formule (III), les différents éléments sont les suivants :

- $M_1$ est choisi dans le groupe des éléments de la colonne 1A et leurs mélanges, de préférence les métaux alcalins tels que le lithium, le sodium, le potassium, le rubidium et le césium,
- $M_2$ est choisi dans le groupe des éléments de la colonne 2A et leurs mélanges, de préférence les métaux alcalino-terreux tels que le béryllium, le magnésium, le calcium, le strontium et le baryum,
- $M_3$ est choisi dans le groupe des terres rares trivalentes choisies parmi les lanthanides, l'yttrium le scandium et leurs mélanges, de préférence les lanthanides tels que le lanthane, le cérium, le praséodyme, le néodyme, le samarium, l'europium, le gadolinium, le terbium, le dysprosium, l'holmium, l'erbium, le thulium, l'ytterbium et le lutécium.

En général, M est constitué d'au plus trois éléments et ce pour des raisons de commodités pour les mêmes raisons, il peut être économiquement intéressant d'utiliser des mélanges commerciaux de terres rares sous quelle que forme qu'ils soient dès lors qu'ils conduisent aisément au composé selon l'invention ; ainsi bien que cela ne soit guère critique, il est fréquent que $\gamma$ soit peu différent de un ($O{,}9 \pm 0{,}1$) et que M ne comporte qu'un métal aux impuretés près.

Im correspond à un composé d'imprégnation constitué d'un métal choisi parmi les alcalins et alcalino-terreux, de préférence, les métaux alcalins, et leurs mélanges associés à un ou plusieurs contre-anions pour assurer la neutralité éléctrique.

Le(s) contre-anion(s) initial(aux), c'est-à-dire avant traitement thermique sont choisis de préférence parmi les anions nitrate, sulfate, chlorure, fluorure, hydrogénophosphate, phosphate, hydrogénosulfate, sulfate, oxalate, acétate, benzoate, etc...

Les anions peuvent être d'une seule espèce ou être constitués d'un mélange des espèces ; pour des raisons de simplicité on préfère n'avoir qu'une seule espèce ou qu'une seule famille d'espèces.

La teneur du catalyseur en agent dopant est généralement telle que le pourcentage pondéral de l'agent dopant exprimé en élément alcalin et/ou alcalino-terreux, par rapport au phosphate de terre trivalente sec est compris entre 0 et 25 %, de préférence entre 3 et 15%.

Une première variante du procédé de l'invention consiste à faire appel à un orthophosphate de terre rare trivalente non dopé. Le catalyseur répond alors à la formule (III) dans laquelle $\alpha$ et $\beta$ sont égaux à 0. Dans ce cas, et comme précisé ultérieurement, une variante préférée consiste à mettre en oeuvre un catalyseur qui a subi au préalable une calcination à haute température.

Parmi les catalyseurs susceptibles d'être mis en oeuvre dans le procédé de l'invention, on peut faire appel à une première famille qui regroupe les orthophosphates de terres rares légères également dénommées terres rares cériques incluant les éléments La, Ce, Pr, Nd, Sm, Eu. Lesdits orthophosphates sont dimorphiques. Ils présentent une structure

hexagonale et évoluent vers une structure monoclinique lorsqu'ils sont chauffés vers une température de 600 - 800°C.

Une deuxième famille d'orthophosphates de terres rares convenant à l'invention regroupent les orthophosphates de Gd, Tb et Dy. Ceux-ci présentent la même structure que les orthophosphates de terres rares cériques mais présentent en plus, une troisième phase cristalline de structure quadratique à haute température (vers 1700°C).

Une troisième famille d'orthophosphates de terres rares regroupent les orthophosphates de terres rares lourdes appelées également terres rares yttriques incluant Y, Ho, Er, Tm, Yb et Lu. Lesdits composés cristallisent uniquement sous la forme quadratique

Parmi les différentes classes d'orthophosphates de terres rares précitées, on fait appel préférentiellement aux orthophosphates de terres rares légères.

On ne sortira pas du cadre de la présente invention à faire appel à tout composé oxygéné du phosphore qui, lors de la synthèse du catalyseur ou lors de la réaction, conduit à un orthophosphate d'un métal de terre rare.

Les orthophosphates des métaux de terres rares de départ, mis en oeuvre dans le procédé de l'invention sont des produits connus. On peut faire appel aux phosphates du commerce, notamment à l'orthophosphate de lanthane ou bien les synthétiser selon les procédés décrits dans la littérature.

Si l'on se réfère aux techniques générales de fabrication de phosphates [notamment au "PASCAL P. Nouveau traité de chimie minérale, tome X (1956), pp.821-823" et "Gmelins Handbuch der anorganischen Chemie (8ième édition) vol.16 (C), pp. 202-206 (1965)"], on peut distinguer deux voies principales d'accès aux phosphates : d'une part, la précipitation d'un sel soluble du métal (chlorure, nitrate) par l'hydrogénophosphate d'ammonium ou l'acide phophorique: d'autre part, la réaction de l'oxyde de métal avec de l'acide phosphorique à chaud ; Dans les deux cas, on peut envisager un traitement de finition avec un hydroxyde alcalin.

Les phosphates desdits métaux peuvent être également préparés par chamottage (réaction solide-solide) de leurs sels avec des sels de phosphore, puis calcination.

On peut se référer, plus particulièrement, pour la préparation :

- de l'orthophosphate de cérium, aux travaux de FUKUO et al [Nippon Kagakkai Shi (Revue de l'Association japonaise de Chimie (4), pp.622-626 (1975)],
- de l'orthophosphate de lanthane, à l'article J.M. COWLEY et al [Journal of Catalysis, 56, pp.185-194 (1979)],
- de l'orthophosphate d'yttrium à la publication de L.S. ESHCHENKO et al [Russian Journal of Inorganic Chemistry, 30, (6), (1985)].

Le produit est ensuite séché selon les techniques classiques connues de l'Homme du Métier. Il se fait avantageusement entre 50°C et 200°C, pendant une durée allant de préférence, de 2 à 8 heures, sous atmosphère normale ou sous pression réduite (par exemple 10 mm de mercure = 1300 Pa) ou par lyophilisation.

Le produit séché peut être ensuite calciné à une température comprise entre 200°C et 1000°C, de préférence entre 400°C et 600°C, pendant une durée variant de 1 à 15 heures, de préférence de 3 à 6 heures.

Le produit peut servir directement de catalyseur, la calcination a lieu à la température et dans les conditions de la réaction.

Lorsque l'on fait appel, selon le procédé de l'invention, à un orthophosphate d'un métal de terre rare trivalente, non dopé, un mode préféré de l'invention consiste à calciner le catalyseur de l'invention, à haute température avant sa mise en oeuvre, par exemple à une température allant de 500°C à 1000°C, de préférence à une température comprise entre 700°C et 800°C, pendant une durée variant de 1 à 15 heures, de préférence de 3 à 6 heures.

En effet, on a constaté de manière inattendue, que la mise en oeuvre d'un catalyseur calciné à haute température, permettait d'améliorer le taux de transformation du phénol et la sélectivité de la réaction.

Une variante préférée de l'invention consiste à faire appel à un orthophosphate de terre rare trivalente dopé par un métal alcalin ou un métal alcalino-terreux.

Les catalyseurs mis en oeuvre dans le procédé de l'invention sont aussi des produits connus car décrits dans la demande de brevet EP-A 0 440 555. Pour leur préparation, on pourra se référer à la demande de brevet EP-A 0 440 555 qui est incorporée par référence à la présente demande.

Un mode de fabrication desdits composés consiste à réaliser l'imprégnation d'un composé de formule $MPO_4$, M ayant été défini antérieurement avec une solution d'imprégnant Imp tel que défini, dans un solvant volatil, de préférence l'eau.

Le produit $MPO_4$ peut être chimiquement modifié par imprégnation à sec ou voie humide.

Ainsi, un mode de préparation consiste à effectuer l'imprégnation à sec de l'orthophosphate métallique $MPO_4$, à l'aide d'une solution d'au moins un sel de métal alcalin ou alcalino-terreux. Comme mentionné précédemment, les contre-anions préférés sont l'hydrogénophosphate ou le phosphate, de préférence l'hydrogénophosphate de césium.

L'imprégnation est réalisée à sec c'est-à-dire que le volume total de solution utilisée est approximativement égal au volume poreux total présenté par l'orthophosphate du métal de terre rare trivalente. On sèche et calcine le produit obtenu.

Plus précisément, l'imprégnation à sec consiste à ajouter à une masse $m_1$ d'une poudre du produit à imprégner un volume V d'une solution aqueuse d'un ou des sels de cations ou d'anions à fixer sur la surface du solide. Le volume V de solution est choisie tel que $V/m_1$ soit égal au volume poreux à l'eau du solide à imprégner.

La concentration C en cations ou anions de la solution imprégnante est choisie telle que le rapport $CVM_2/m_1$ soit égal au pourcentage en poids choisi d'espèce imprégnante fixée sur la surface du produit à imprégner (avec $M_2$ = masse molaire de l'espèce imprégnante). On procède goutte à goutte à l'ajout de la solution de manière à obtenir une adsorption homogène.

Le produit peut ensuite être laissé au repos pendant un temps variable à température ambiante. Le produit est ensuite séché suivant les techniques classiques connues de l'Homme du Métier. Le séchage est généralement conduit à la pression atmosphérique ou sous pression réduite ou lyophilisation. Il peut être également calciné.

L'imprégnation voie humide se fait en dispersant l'orthophosphate d'un métal de terre rare trivalente, dans une solution aqueuse de sels de cations et/ou d'anions à fixer sur la surface du solide.

Cette solution peut présenter une concentration variant de $10^{-3}$ M à 10 M en l'espèce imprégnante.

Le pH de la solution pourra être avantageusement ajusté à une valeur au moins égale à celle du point isoélectrique du produit à modifier pour fixer préférentiellement les cations (cas usuel) ; toutefois, cette condition n'est pas impérative. Il est possible, au-dessous de ce point isoélectrique, de fixer correctement les cations lorsque les anions associés ont un caractère très "covalent" comme les sulfates et les phosphates.

La température de la solution peut varier de l'ambiante à 100°C.

La dispersion est agitée vigoureusement, pendant un temps variable.

Le produit est ensuite filtré et éventuellement lavé.

Dans ces deux variantes de préparation desdits composés chimiques, on précisera que le séchage est conduit avantageusement à une température variant de 50°C à 200°C, pendant une durée allant de préférence, de 2 à 8 heures.

En ce qui concerne l'opération de calcination, celle-ci est conduite à une température comprise entre 200°C et 1000°C, de préférence entre 400°C et 700°C, pendant une durée variant de 1 à 15 heures, de préférence de 3 à 6 heures.

Les catalyseurs préférés de l'invention répondent à la formule (III) dans laquelle M est le lanthane, le cérium et le samarium éventuellement dopé par un métal alcalin, de préférence le césium.

Les catalyseurs selon la présente invention sont tels que la surface des corps catalytiques soit formée pour partie au moins d'un composé selon la présente invention.

La phase catalytique peut être utilisée pure ou être supportée. Dans la suite de la description, on désignera par corps catalytique, le catalyseur dans sa forme particulaire unitaire, que le catalyseur soit supporté ou non. Le dépôt de la phase catalytique sur le support est faite selon des techniques connues de l'Homme du métier.

Ces corps catalytiques peuvent être de toute forme en elles-mêmes connues pour des catalyseurs solides utilisables en phase gazeuse.

Le reste dudit corps catalytique, c'est-à-dire pour l'essentiel la partie n'entrant pas en contact avec le mélange gazeux réactionnel peut être en matériau(x) quelconque(s), pourvu qu'il(s) soi(en)t inerte(s) dans les conditions d'usage ; pour des raisons de facilité de fabrication, il peut être réalisé en composés choisis dans le groupe constitué par les phosphates, les hydrogénophosphates et leur mélange. Les catalyseurs peuvent être également réalisés entièrement en composés chimiques (III) selon la présente invention.

Le catalyseur peut se présenter sous différentes formes : poudre, ou produits mis en forme tels que granulés (par exemple cylindres), billes, pastilles ou monolithes (blocs en forme de nid d'abeilles) qui sont obtenus par extrusion, moulage, compactage ou tout autre type de procédé connu.

La surface spécifique du catalyseur est la plus élevée possible, en général au moins égal à 1 $m^2$, avantageusement au moins égal à 10 $m^2$, le plus souvent entre 50 et 150 $m^2/g$, de préférence entre 50 et 100 $m^2/g$.

Conformément au procédé de l'invention, la réaction de O-alkylation est conduite en phase vapeur, en mettant en contact le composé phénolique de formule (I) avec le catalyseur de l'invention.

La quantité d'alcanol utilisée est égale ou supérieure à la quantité stoechiométrique nécessaire pour éthérifier un ou plusieurs groupements hydroxyle en groupements alkoxy (ou aralkoxy).

Généralement, l'alcanol est mis en oeuvre en une quantité telle que le rapport entre le nombre de fonctions hydroxyle apportées par l'alcanol et le nombre de fonctions hydroxyle du composé phénolique de formule (I) varie entre 0,5 et 500, de préférence entre 1 et 5.

En ce qui concerne le catalyseur, sa productivité pondérale et horaire est comprise entre 0,1 et 20 $h^{-1}$, de préférence entre 1 et 5 $h^{-1}$: la productivité pondérale horaire d'un catalyseur étant définie par le rapport pondéral entre le composé phénolique introduit par heure et le catalyseur.

On rappellera qu'il est préférable de calciner le catalyseur, à haute température, avant sa mise en oeuvre lorsque l'on fait appel à un orthophosphate de terre rare non dopé.

Le gaz vecteur est optionnel et est en général un gaz ou un mélange de gaz non réactifs dans les conditions de la réaction. On peut faire appel à des gaz tels que l'azote, l'air, l'argon ou l'hélium. Avantageusement, le rapport volumique entre le gaz vecteur et le composé phénolique varie entre 0 et 10, de préférence entre 0,1 et 2,0.

La température de la réaction de O-alkylation est généralement comprise entre 150°C et 500°C, et, de préférence de 200°C et 350°C.

La pression réactionnelle peut être comprise entre $10^{-2}$ et 50 bars, de préférence la pression atmosphérique.

Conformément au procédé de l'invention, on vaporise les réactifs de départ à savoir le composé phénolique et l'alcanol. Ils sont introduits au contact du catalyseur, de préférence entraînés par un gaz vecteur.

Le temps de contact qui est défini comme le rapport entre le volume apparent de catalyseur et le débit du flux gazeux (qui inclut le gaz vecteur), peut varier largement, et est le plus souvent compris entre 0,5 et 100 secondes. Le temps de contact est choisi de préférence entre 1 et 10 secondes.

Une variante préférée du procédé de l'invention consiste à ajouter de l'eau qui est également vaporisée. Il est souhaitable que ladite quantité ne soit pas trop élevée. Ainsi le rapport molaire entre l'eau et le composé phénolique est avantageusement compris entre 0 et 5, et de préférence entre 0,1 et 1.

En ce qui concerne la réalisation pratique de l'invention, on commence à préparer le lit catalytique qui est constitué par la phase active catalytique déposée sur un support (par exemple, verre fritté) ce qui permet la circulation des gaz sans élution du catalyseur. Ensuite, les. réactifs sont mis en oeuvre et plusieurs variantes sont possibles.

Il est possible de vaporiser chacun des réactifs, composé phénolique, alcanol et éventuellement eau, dans des chambres différentes, puis d'effectuer le mélange dans une chambre de mélange et d'introduire le flux gazeux résultant sur le catalyseur. Le gaz vecteur peut être introduit en parallèle audit flux gazeux ou bien au niveau de la chambre de mélange.

Une autre variante consiste à préparer une solution comprenant le composé phénolique, l'alcanol et éventuellement de l'eau, puis à vaporiser ledit mélange et à l'introduire sur le catalyseur, en parallèle avec le gaz vecteur.

Un autre mode de réalisation pratique de l'invention consiste à faire fondre le composé phénolique en le chauffant jusqu'à sa température de fusion et l'on fait passer au-dessus un flux gazeux comprenant l'alcanol et l'eau éventuellement. Ce flux se sature en composé phénolique et il est mis alors en contact avec le catalyseur.

Un autre mode d'exécution de l'invention est de faire appel à un solvant organique qui est choisi de telle sorte qu'il solubilise le composé phénolique et l'alcanol mis en oeuvre.

On fait appel de préférence selon l'invention à un solvant aprotique polaire ayant un point d'ébullition élevé supérieur à 80°C et, de préférence, compris entre 80°C et 300°C.

A titre de solvants aprotiques polaires susceptibles d'être mis en oeuvre dans le procédé de l'invention, on peut citer les carboxamides cycliques comme la N-méthylpyrrolidone les nitriles aliphatiques ou aromatiques comme l'acétonitrile, le propionitrile ou le benzonitrile.

Une classe préférée de solvants convenant particulièrement bien à l'invention sont les éthers et, plus précisément, les éthers diméthyliques dérivant de l'oxyde d'éthylène ou de l'oxyde de propylène tels que le diméthyléther de l'éthylèneglycol (ou diméthoxy-1,2 éthane), le diméthyléther du diéthylèneglycol (ou diméthoxy-1,5 oxa-3 pentane), le diméthoxy-1,8 dioxa-3,6 octane, le diméthoxy-1,11 trioxa-3,6,9 undécane, le diméthoxy-1,2 méthyl-1 éthane, le diméthoxy-1,5 diméthyl-1,4 oxa-3 pentane, le diméthoxy-1,7 diméthyl-1,4 dioxa-3,6 octane.

On peut également utiliser plusieurs solvants.

Parmi tous les solvants précités, le diméthyléther de l'éthylèneglycol et le diméthyléther du diéthylèneglycol sont préférés.

La quantité de composé phénolique mis en oeuvre dans le solvant est généralement telle que le rapport molaire solvant/composé phénolique soit compris entre 0 et 20 et, de préférence, entre 0 et 5.

Ainsi, on prépare une solution organique comprenant le composé phénolique, l'alcanol et éventuellement de l'eau, puis on vaporise ledit mélange et on l'introduit sur le catalyseur, en parallèle avec le gaz vecteur.

En fin de réaction, on condense l'ensemble des gaz et l'on sépare les réactifs non réagis et les produits obtenus, par distillation. Il est également possible de séparer ceux-ci, par condensation fractionnée.

Le procédé de l'invention permet donc d'obtenir des composés aromatiques alkoxylés qui répondent à la formule (IV).

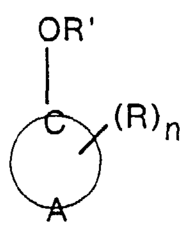

(IV)

dans laquelle :

- A symbolise un reste d'un radical carbocyclique aromatique, monocyclique ou polycyclique ou un radical divalent constitué par un enchaînement de deux ou plusieurs radicaux carbocycliques aromatiques monocycliques,
- R représente un ou plusieurs substituants, identiques ou différents,
- R' représente un radical hydrocarboné ayant de 1 à 24 atomes de carbone, qui peut être un radical aliphatique acyclique saturé ou insaturé, linéaire ou ramifié ; un radical cycloaliphatique saturé, insaturé ou aromatique, monocyclique ou polycyclique un radical aliphatique saturé ou insaturé, linéaire ou ramifié, porteur d'un substituant cyclique,
- n est un nombre inférieur ou égal à 5.

Le reste A pouvant également porter d'autres groupes hydroxyle, il est possible selon le procédé de l'invention de préparer des composés polyalkoxylés.

Le procédé de l'invention est parfaitement bien adapté à la préparation des composés aromatiques mono- ou polyalkoxylés résultant de la condensation :

- des composés phénoliques répondant à la formule (I) suivants :
  - le phénol
  - l'hydroquinone
  - la pyrocatéchine
  - le méthoxy-2 phénol
  - le méthoxy-3 phénol
  - le méthoxy-4 phénol
  - l'éthoxy-2-phénol
  - l'éthoxy-3-phénol
  - l'éthoxy-4 phénol
  - l'aldéhyde salicylique
  - le p-hydroxybenzaldéhyde
  - le salicylate de méthyle
  - l'ester méthylique de l'acide p-hydroxybenzoique
  - le chloro-2 phénol
  - le chloro-4 phénol
  - le nitro-2 phénol
  - le nitro-4 phénol
  - le N-acétyl p-aminophénol
  - la vanilline
  - l'isovanilline
  - l'hydroxy-2 acétamido-5 benzaldéhyde
  - l'hydroxy-2 propionamido-5 benzaldéhyde
  - l'allyloxy-4 benzaldéhyde
  - le dichloro-2,4 phénol
  - le dichloro-2,6 phénol
  - la méthylhydroquinone
  - le pyrogallol
  - la bromo-4 vanilline
  - l'hydroxy-4 vanilline
  - le trichloro-2,4,6 phénol
  - le diméthoxy-3,5 hydroxy-4 benzaldéhyde
  - le bromo-6 naphtol-2
- et les alcanols suivants :
  - le méthanol
  - l'éthanol
  - l'isopropanol
  - l'éthylène glycol
  - le glycérol

- le cyclohexanol
- l'alcool benzylique

Le procédé de l'invention est particulièrement bien adapté à la préparation de l'anisole, du gaïacol, du guétol, du p-méthoxyphénol et de l'éthylène dioxybenzène. Les catalyseurs alors préférés sont l'orthophosphate de lanthane, de cérium ou de samarium éventuellement dopé par du césium.

L'intérêt du procédé, objet de la présente invention est qu'il permet d'obtenir les éthers des composés phénoliques avec un bon taux de transformation du phénol de départ et avec une bonne sélectivité de réaction.

Un autre avantage du procédé de l'invention est que le catalyseur de l'invention présente une stabilité remarquable. Il a donc une très longue durée de vie car il ne se déactive pas.

Dans le cas de la O-méthylation de la pyrocatéchine, le procédé de l'invention est très intéressant car il permet de moduler la production du gaïacol ou du vératrole selon les besoins du marché. Il en est de même pour la O-méthylation de l'hydroquinone qui permet d'obtenir le p-méthoxyphénol et/ou le p-diméthoxybenzène.

Les exemples qui suivent illustrent l'invention sans toutefois la limiter.

**EXEMPLES**

Dans les différents exemples qui suivent, les abréviations TT, RR, RT ont la signification suivante :

$$\text{Taux de transformation : TT} = \frac{\text{nombre de moles de phénol transformé}}{\text{nombre de moles de phénol introduit}} \text{ en \%}$$

$$\text{Rendement Réel : RR} = \frac{\text{nombre de moles d'alkyl-aryl éther formé}}{\text{nombre de moles de phénol introduit}} \text{ en \%}$$

$$\text{Sélectivité : RT} = \frac{\text{nombre de moles d'alkyl-aryl éther formé}}{\text{nombre de moles de phénol transformé}} \text{ en \%}$$

Exemple 1

On introduit dans un réacteur, 106 g de $H_3PO_4$ (à 85% vendu par la société Prolabo) soit 0,92 mole dans 800 ml d'eau désionisée.

On agite à 500-700 tours /minute.

On introduit 299 g de $La_2(CO_3)_3$, $12H_2O$ à froid, (soit 0,8 mole de lanthane dans 736 ml d'eau), lentement et sous forte agitation.

On chauffe ensuite le milieu réactionnel jusqu'à 80°C, pendant deux heures trente.

On laisse refroidir jusqu'à température ambiante sans agitation pendant une demi-heure et on finit dans un bain d'eau froide.

La suspension est filtrée sur un verre fritté n°3 jusqu'à épuisement des eaux-mères

Le produit est alors redispersé dans 2 litres d'eau, sous forte agitation et laissé en suspension pendant une demi-heure, tout en maintenant l'agitation .

La suspension est filtrée sur un verre fritté n°3 jusqu'à épuisement des eaux-mères

Le produit est remis en dispersion dans 900 ml d'eau désionisée et neutralisé par une solution d'ammoniaque jusqu'à un pH égal à 9.

Le produit est filtré, lavé à l'eau et centrifugé avant d'être séché à 110°C.

Exemple 2

On prélève 4,7 ml d'une solution 6 M de CsOH, à laquelle on ajoute 14,12 ml d'une solution 1M de $H_3PO_4$. On ajoute alors la quantité d'eau nécessaire pour compléter à 50 ml.

On prend 50g de produit préparé dans l'exemple 1 que l'on place dans un bécher de 200 ml.

On introduit goutte à goutte 20 ml de la solution d'imprégnation en écrasant les agglomérats formés et en homogénéïsant bien.

On laisse le produit reposer une heure. On le séche une nuit à 110°C et on le calcine 2 heures à 500°C.

Teneur en césium sur sec = 3%

Exemple 3

Dans un réacteur tubulaire en quartz ayant un diamètre intérieur de 10 mm, on charge sur verre fritté, 3 ml de catalyseur phosphate de lanthane préparé selon l'exemple 2 (soit 3,0 g). Le catalyseur est surmonté d'un lit de billes de verre dont le rôle est d'homogénéïser le flux gazeux, avant contact avec le catalyseur. On introduit en tête de réacteur, en parallèle, de l'azote à l'aide d'un débimètre Brooks et une solution de phénol dans le méthanol à l'aide d'un pousse seringue.

A la sortie du réacteur, les effluents gazeux sont condensés dans deux pièges en série, le premier refroidi par un bain eau-glace, le second refroidi par un bain acétone-carboglace. Les piégats sont regroupés, dilués puis analysés par chromatographie liquide haute performance (CLHP).

La réaction est conduite en utilisant un débit d'azote de 1,2 l/h et un débit d'alimentation de la solution de 6,4 ml (liquide) par heure. Le rapport molaire méthanol/phénol est de 10.

Après 1 heure de réaction à 300°C, on observe les performances suivantes :

- $TT_{phénol}$=10,6 %
- $RR_{anisole}$=10,0 %
- $RT_{anisole}$=94,3 %

La réaction est également conduite 1 heure à 330°C et à 360°C, avec les performances suivantes :

| | Phénol | Anisole | |
|---|---|---|---|
| Température (°C) | TT (%) | RR (%) | RT (%) |
| 330 | 22,7 | 21,7 | 90,87 |
| 360 | 52,7 | 48,6 | 89,0 |

Exemple 4

On reproduit l'exemple 3 à la différence près que l'on utilise 2,5 ml de catalyseur $LaPO_4$ préparé selon l'exemple 1 correspondant à 2,0 g.

Une solution de pyrocatéchine dans le méthanol (rapport molaire méthanol/pyrocatéchine = 10) est injectée à un débit de 5ml/h.

Après 1 heure de réaction à 270°C, on observe les performances suivantes :

- $TT_{pyrocatéchine}$ = 60 %
- $RR_{gaïacol}$= 59 %
- $RT_{gaïacol}$ = 98,3 %

Les mêmes conditions sont mises en oeuvre à une température de 330°C, les performances suivantes sont alors observées :

- $TT_{pyrocatéchine}$ = 88,1 %
- $RR_{gaïacol}$ = 56,6 %
- $RT_{gaïacol}$ = 64,2 %
- vératrole : traces

Exemple 5

On procède comme dans l'exemple 2 à l'aide d'une solution d'imprégnation préparée à partir de 11 ml d'une solution 6M de CsOH et 33 ml d'une solution 1M de $H_3PO_4$.

La teneur en césium sur sec est de 7%

Exemple 6

On procède comme dans l'exemple 2 à l'aide d'une solution d'imprégnation préparée à partir de 15,67 ml d'une solution 6M de CsOH et 23,5 ml d'une solution 2M de $H_3PO_4$.

La teneur en césium sur sec est de 10%

Exemple 7

La réaction est conduite dans des conditions identiques à celles de l'exemple 4 en utilisant 2,5 ml du catalyseur obtenu à l'exemple 6.

Après 1 heure de réaction à 270°C :

- $TT_{pyrocatéchine}$ = 40,5 %
- $RR_{gaïacol}$ = 40 %

Les mêmes conditions sont mises en oeuvre pour réaliser la réaction à 300°C, 330°C et 360°C. Les résultats, pour une heure à chacune de ces températures sont donnés dans le tableau suivant :

| Température (°C) | Pyrocatéchine | Gaïacol | | Vératrole | |
|---|---|---|---|---|---|
| | TT (%) | RR (%) | RT (%) | RR (%) | RT (%) |
| 300 | 52,2 | 51,0 | 97,8 | Traces | - |
| 330 | 85,7 | 71,0 | 82,3 | 8,0 | 9,7 |
| 360 | 97,0 | 62,6 | 64,5 | 25,1 | 25,9 |

Exemple 8

La réaction est conduite dans des conditions identiques à celles de l'exemple 4 en utilisant 2,5 ml du catalyseur obtenu à l'exemple 5.

Après 1 heure de réaction à 270°C :

TT pyrocatéchine = 26,6 %
RR gaiacol = 26,6 %

Les mêmes conditions sont mises en oeuvre pour réaliser la réaction à 300°C, 330°C et 360°C. Les résultats, pour une heure à chacune de ces températures sont données dans le tableau suivant :

| Température (°C) | Pyrocatéchine | Gaïacol | | Vératrole | |
|---|---|---|---|---|---|
| | TT (%) | RR (%) | RT (%) | RR (%) | RT (%) |
| 300 | 61,2 | 58,4 | 95,5 | 2,6 | 4,2 |
| 330 | 86,7 | 69,6 | 80,3 | 17,0 | 19,5 |
| 360 | 94,2 | 67,2 | 71,4 | 26,8 | 28,5 |

Exemple 9

On reproduit la synthèse décrite dans l'exemple 1 en substituant $La_2(CO_3)_3$ par $Ce_2(CO_3)_3$ (mole par mole).

Exemple 10

On introduit 91,2 g d'$H_3PO_4$ (à 85% vendue par la société Prolabo) et 340 ml d'eau dans un réacteur de 2 litres.

On agite à 500-700 tours/minute. On chauffe jusqu'à atteindre 90°C.

On ajoute 140,93 g de $Sm_2O_3$ (provenant de la calcination à 700°C, de $Sm_2(CO_3)_3$ commercialisé par Rhône-Poulenc) par portions, en 30 à 40 minutes sous agitation (2 à 3 spatules à la fois toutes les 4 à 5 minutes).

La température du mélange réactionnel est maintenue entre 87 et 93°C, pendant 3 heures.

Le produit est alors filtré, lavé sur verre fritté 3 fois et séché à 110°C une nuit.

Exemple 11

On imprègne 10g du produit préparé à l'exemple 10 avec 11,7 ml des 50 ml d'une solution de CsOH et $H_3PO_4$ préparée à partir de 5,16 ml de CsOH 3M et 7,74 ml de $H_3PO_4$ 1M, suivant la procédure de l'exemple 2.

Exemples 12 et 13

La réaction est conduite dans des conditions identiques à celles de l'exemple 4. On met en oeuvre 2,5 ml de $CePO_4$ (exemple 12) et $SmPO_4$ (exemple 13).

La préparation de $CePO_4$ est décrite dans l'exemple 9 alors que $SmPO_4$ est préparé selon le mode opératoire décrit dans l'exemple 10.

Les résultats obtenus pour 1 heure de fonctionnement à 270°C et 360°C sont donnés dans le tableau suivant :

| | Température 270°C | | | Température 360°C | | |
|---|---|---|---|---|---|---|
| Catalyseur | TT (%) PC | RT (%) Gaïacol | RT (%) Vératrole | TT (%) PC | RT (%) Gaïacol | RT (%) Vératrole |
| $CePO_4$ | 60,5 | 92,0 | 4,6 | 95,4 | 2,4 | 6,2 |
| $SmPO_4$ | 87 | 93 | 6,5 | 97,5 | 12 | 4,7 |

Exemple 14

La réaction est conduite dans des conditions identiques à celles de l'exemple 4 en utilisant 2,5 ml du catalyseur préparé dans l'exemple 11.

Les performances pour 1 heure de fonctionnement à diverses températures sont données dans le tableau suivant:

| | Pyrocatéchine | Gaïacol | | Vératrole | |
|---|---|---|---|---|---|
| Température (°C) | TT (%) | RR (%) | RT (%) | RR (%) | RT (%) |
| 270 | 14,1 | 14,1 | 100 | - | - |
| 300 | 33,0 | 32,9 | 99,7 | - | - |
| 330 | 64,6 | 64,5 | 99,8 | - | - |
| 360 | 91,2 | 87,1 | 95,5 | 3,9 | 4,3 |
| 390 | 97,5 | 86,8 | 89 | 10,5 | 10,8 |

Exemple 15

Le catalyseur de l'exemple 6 est utilisé dans les conditions suivantes :

- Température : 300 °C
- Débit d'azote : 0,5 l/h
- Débit de la solution de pyrocatéchine dans le méthanol :5 ml/h.
- Rapport molaire méthanol/pyrocatéchine: 10

Les performances sont observées à diverses durées de réaction :

| | | |
|---|---|---|
| 2 heures | TT = 42,3 % | $RR_{gaïacol}$ = 42,5 % |
| 3 " | TT = 42,8 % | $RR_{gaïacol}$ = 42,8 % |
| 4 " | TT = 44,7 % | $RR_{gaïacol}$ = 42,0 % |
| 6 " | TT = 42,6 % | $RR_{gaïacol}$ = 42,5 % |

Exemple 16

Une solution de pyrocatéchine dans l'éthylène glycol est mise en réaction en utilisant le même catalyseur que celui décrit dans l'exemple 6. Les conditions suivantes sont utilisées :

- 2,5 ml de catalyseur (2,6g)
- Débit d'azote : 1,2 l/h
- Débit de la solution de pyrocatéchine dans l'éthylène glycol : 2,9 ml/h

- Rapport molaire éthylène glycol/pyrocatéchine : 10

Après 1 heure de réaction à 330 °C, on observe les performances suivantes :
- TT = 100 %

Le seul produit observé par chromatographie en phase gazeuse (CPG) et par CLPH est l'éthylène dioxybenzène.

A 270°C et 300 °C, un produit intermédiaire est obtenu dont la structure détérminée par Spectrographie de masse est :

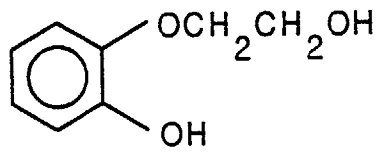

Exemple 17

Une solution d'hydroquinone, de méthanol et d'eau (rapport molaire : hydroquinone/eau/méthanol = 1/7/16) est injectée à 330°C à un débit de 2,8 ml/h sur un lit de catalyseur (2,5 ml correspondant à 2,57 g) préparé selon l'exemple 6. Le débit d'azote est de 1,2 l/h.

Après une heure de réaction, on observe les performances suivantes :

- TT = 25,8 %
- $RR_{\text{p-méthoxyphénol}}$ = 24,2%
- $RT_{\text{p-méthoxyphénol}}$ = 94,1 %

Le principal sous-produit observé est la méthylhydroquinone. On n'observe que des traces de p-diméthoxybenzène.

Exemple 18

On introduit dans un réacteur, 106 g de $H_3PO_4$ (à 85% vendu par la société Prolabo) soit 0,92 mole dans 800 ml d'eau désionisée.

On agite à 500-700 tours /minute.

On introduit 299 g de $La_2(CO_3)_3$, $12H_2O$ à froid, (soit 0,8 mole de lanthane dans 800 ml d'eau), lentement et sous forte agitation.

On chauffe ensuite le milieu réactionnel jusqu'à 80°C, pendant deux heures trente.

On laisse refroidir jusqu'à température ambiante sans agitation pendant une demi-heure et on finit dans un bain d'eau froide.

La suspension est filtrée sur un verre fritté n°3 jusqu'à épuisement des eaux-mères

Le produit est alors redispersé dans 2 litres d'eau, sous forte agitation et laissé en suspension pendant une demi-heure, tout en maintenant l'agitation .

Cette opération de lavage est effectuée deux fois suivant la même procédure.

Le produit ainsi lavé est séché à 110°C.

Exemple 19

Le produit préparé comme décrit dans l'exemple 18 est calciné 3 heures à 700°C.

Exemple 20

On introduit dans un réacteur, 182,4 g de $H_3PO_4$ (à 85% vendu par la société Prolabo) soit 1,6 mole dans 640 ml d'eau désionisée.

On chauffe le milieu réactionnel jusqu'à 85°C et quand la température est atteinte, on introduit 260,8 g de $La_2O_3$ (soit 0,8 mole de lanthane) de façon régulière (14 g toutes les 5 minutes).

On laisse chauffer le mélange réactionnel 1 heure à 85°C, puis on laisse refroidir jusqu'à température ambiante sous agitation.

Le produit est récupéré par centrifugation (3600 tours/mn), puis redispersé dans 400 ml d'eau désionisée.

Le produit ainsi lavé est récupéré par centrifugation.
Cette opération de lavage est effectuée 3 fois suivant la même procédure.
Le produit ainsi lavé est séché à 110°C.

Exemple 21

Le produit préparé comme décrit dans l'exemple 20 est calciné 3 heures à 700°C.

Exemple 22

La réaction est conduite dans les conditions identiques à celles de l'exemple 3.
Le catalyseur de l'exemple 18 est utilisé dans les conditions suivantes :

. Catalyseur : 3,5 g
. Calcination : 2 h à 400°C, sous azote (2,2 l/h)
. Réaction:

Température : 330°C
Débit d'azote : 2,2 l/h

Une solution de pyrocatéchine dans 3 équivalents d'éthanol est introduite à un débit de 6,2 g/h.
Après 1 heure de réaction, on observe les performances suivantes :

- $TT_{pyrocatéchine}$ = 31 %
- $RT_{guétol}$ = 56 %

Exemple 23

Le catalyseur de l'exemple 19 est utilisé dans les conditions de l'exemple 22.
Après 1 heure de réaction, on observe les performances suivantes :

- $TT_{pyrocatéchine}$ = 33%
- $RT_{guétol}$ = 90 %

Exemple 24

Le catalyseur de l'exemple 20 est utilisé dans les conditions de l'exemple 22.
Après 1 heure de réaction, on observe les performances suivantes :

- $TT_{pyrocatéchol}$ = 54%
- $RT_{guétol}$ = 55 %

Exemple 25

Le catalyseur de l'exemple 21 est utilisé dans les conditions de l'exemple 22.
Après 1 heure de réaction, on observe les performances suivantes :

- $TT_{pyrocatéchol}$ = 40%
- $RT_{guétol}$ = 80 %

**Revendications**

**1.** Procédé de O-alkylation d'un composé phénolique caractérisé par le fait qu'il consiste à faire réagir ledit composé phénolique avec un alcanol, en phase gazeuse, en présence d'une quantité efficace d'un catalyseur choisi parmi les orthophosphates de métaux de terres rares trivalentes.

**2.** Procédé selon la revendication 1 caractérisé par le fait que le composé phénolique répond à la formule générale (I) :

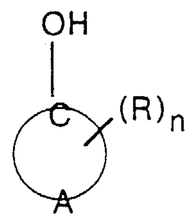

(I)

dans laquelle :

- A symbolise un reste d'un radical carbocyclique aromatique, monocyclique ou polycyclique ou un radical divalent constitué par un enchaînement de deux ou plusieurs radicaux carbocycliques aromatiques monocycliques,
- R représente un ou plusieurs substituants, identiques ou différents,
- n est un nombre inférieur ou égal à 5.

avec un alcool répondant à la formule générale (II) :

$$R' - OH \qquad (II)$$

dans ladite formule (II) :

- R' représente un radical hydrocarboné ayant de 1 à 24 atomes de carbone, qui peut être un radical aliphatique acyclique saturé ou insaturé, linéaire ou ramifié ; un radical cycloaliphatique saturé ou insaturé, monocyclique ou polycyclique un radical aliphatique saturé ou insaturé, linéaire ou ramifié, porteur d'un substituant cyclique.

**3.** Procédé selon l'une des revendications 1 et 2 caractérisé par le fait que le composé phénolique répond à la formule (I) dans laquelle le ou les radicaux R représentent l'un des groupes suivants :

. un atome d'hydrogène,
. un radical alkyle, linéaire ou ramifié, ayant de 1 à 6 atomes de carbone, de préférence de 1 à 4 atomes de carbone,
. un radical alcényle linéaire ou ramifié ayant de 2 à 6 atomes de carbone, de préférence, de 2 à 4 atomes de carbone,
. un radical alkoxy linéaire ou ramifié ayant de 1 à 6 atomes de carbone, de préférence de 1 à 4 atomes de carbone,
. un groupe acyle ayant de 2 à 6 atomes de carbone,
. un radical de formule :

$$-R_1-OH$$

$$-R_1-COOR_2$$

$$-R_1-CHO$$

$$-R_1-NO_2$$

$$-R_1-CN$$

$$-R_1-(NR_2)_2$$

$-R_1-CO-(NR_2)_2$

$-R_1-X$

$-R_1-CF_3$

dans lesdites formules, $R_1$ représente un lien valentiel ou un radical hydrocarboné divalent, linéaire ou ramifié, saturé ou insaturé, ayant de 1 à 6 atomes de carbone ; $R_2$ représente un atome d'hydrogène ou un radical alkyle linéaire ou ramifié ayant de 1 à 6 atomes de carbone ; X symbolise un atome d'halogène, de préférence un atome de chlore, de brome ou de fluor.

**4.** Procédé selon l'une des revendications 1 à 3 caractérisé par le fait que le composé phénolique répond à la formule (I) dans laquelle :

- R représente :

  . un atome d'hydrogène
  . un groupe OH,
  . un radical alkyle linéaire ou ramifié ayant de 1 à 6 atomes de carbone,
  . un radical alcényle linéaire ou ramifié ayant de 2 à 6 atomes de carbone,
  . un radical alkoxy linéaire ou ramifié ayant de 1 à 6 atomes de carbone,
  . un groupe -CHO,
  . un groupe acyle ayant de 2 à 6 atomes de carbone,
  . un groupe $-COOR_2$ où $R_2$ a la signification donnée précédemment,
  . un groupe $-NO_2$,
  . un atome d'halogène, de préférence, de fluor, de chlore, de brome,
  . un groupe $-CF_3$.

- n est un nombre égal à 0, 1, 2 ou 3.

**5.** Procédé selon l'une des revendications 1 à 4 caractérisé par le fait que le composé phénolique répond à la formule (I) dans laquelle le reste A représente :

- un radical carbocyclique aromatique monocyclique ou polycyclique avec des cycles pouvant former entre eux un système orthocondensé répondant à la formule (Ia) :

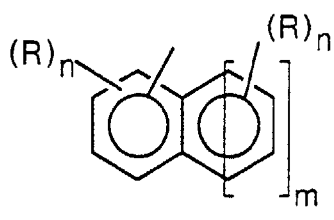
(Ia)

dans ladite formule (Ia), m représente un nombre égal à 0, 1 ou 2 et les symboles R et n identiques ou différents ayant la signification donnée précédemment,

- un radical constitué par un enchaînement de deux ou plusieurs radicaux carbocycliques aromatiques monocycliques répondant à la formule (Ib) :

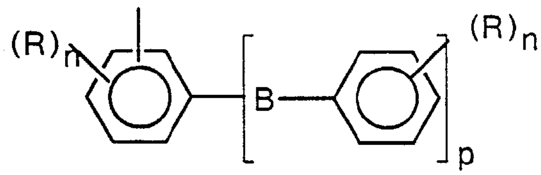

(Ib)

dans ladite formule (Ib), les symboles R et n identiques ou différents ont la signification donnée précédemment, p est un nombre égal à 0, 1, 2 ou 3 et B représente:

- un lien valentiel
- un radical alkylène ou alkylidène ayant de 1 à 4 atomes de carbone, de préférence, un radical méthylène ou isopropylidène,
- un des groupes suivants :
  -O-, -CO-, -COO- , -OCOO-,
  -S-, -SO-, $SO_2$-,

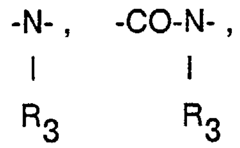

dans ces formules, $R_3$ représente un atome d'hydrogène ou un radical alkyle ayant de 1 à 4 atomes de carbone, un radical cyclohexyle ou phényle.

**6.** Procédé selon la revendication 5 caractérisé par le fait que le composé phénolique de formule (I) présente un reste A qui répond aux formules (Ia) et (Ib) dans lesquelles :

- R représente un atome d'hydrogène, un groupe hydroxyle, un groupe _CHO, un groupe -$NO_2$, un radical alkyle ou alkoxy linéaire ou ramifié ayant de 1 à 6 atomes de carbone, de préférence, de 1 à 4 atomes de carbone,
- B symbolise un lien valentiel, un radical alkylène ou alkylidène ayant de 1 à 4 atomes de carbone ou un atome d'oxygène,
- m est égal à 0 ou 1,
- n est égal à 0, 1 ou 2,
- p est égal à 0 ou 1.

**7.** Procédé selon l'une des revendications 1 à 6 caractérisé par le fait que le composé phénelique de formule (I) est choisi parmi :

- le phénol
- l'hydroquinone
- la pyrocatéchine
- le méthoxy-2 phénol
- le méthoxy-3 phénol
- le méthoxy-4 phénol
- l'éthoxy-2-phénol
- l'éthoxy-3-phénol
- l'éthoxy-4 phénol
- l'aldéhyde salicylique
- le p-hydroxybenzaldéhyde
- le salicylate de méthyle
- l'ester méthylique de l'acide p-hydroxybenzoique
- le chloro-2 phénol
- le chloro-4 phénol
- le nitro-2 phénol

- le nitro-4 phénol
- le N-acétyl p-aminophénol
- la vanilline
- l'isovanilline
- l'hydroxy-2 acétamido-5 benzaldéhyde
- l'hydroxy-2 propionamido-5 benzaldéhyde
- l'allyloxy-4 benzaldéhyde
- le dichloro-2,4 phénol
- le dichloro-2,6 phénol
- la méthylhydroquinone
- le pyrogallol
- la bromo-4 vanilline
- l'hydroxy-4 vanilline
- le trichloro-2,4,6 phénol
- le diméthoxy-3,5 hydroxy-4 benzaldéhyde
- le bromo-6 naphtol-2

**8.** Procédé selon la revendication 2 caractérisé par le fait que l'alcanol répond à la formule générale (II) dans laquelle R' représente un radical alkyle, alcényle, alcadiényle, alcynyle, linéaire ou ramifié ayant de préférence de 1 à 24 atomes de carbone ; la chaîne hydrocarbonée pouvant être éventuellement : - interrompue par l'un des groupes suivants :

-O-, -CO-, -COO-, -OCOO-, -S-, $-SO_2-$,

$$\begin{array}{cc} -N- , & -CO-N- , \\ | & | \\ R_4 & R_4 \end{array}$$

dans ces formules $R_4$ représente l'hydrogène ou un radical alkyle linéaire ou ramifié ayant de 1 à 4 atomes de carbone, de préférence, un radical méthyle ou éthyle,

- et/ou porteuse de l'un des substituants suivants :
  -OH, -OCOO-, $-COOR_4$, -CHO, $-NO_2$, -X, $-CF_3$ dans ces formules, $R_4$ ayant la signification donnée précédemment.

**9.** Procédé selon la revendication 2 caractérisé par le fait que l'alcanol répond à la formule générale (II) dans laquelle R' représente un reste aliphatique acyclique, saturé ou insaturé, linéaire ou ramifié porteur d'un substituant cyclique éventuellement substitué ledit cycle pouvant être relié au cycle par un lien valentiel ou par l'un des groupes suivants :
-O-, -CO-, -COO-, -OCOO-, -S-, $-SO_2-$,

$$\begin{array}{cc} -N- , & -CO-N- , \\ | & | \\ R_4 & R_4 \end{array}$$

dans ces formules $R_4$ ayant la signification donnée précédemment dans la revendication 8.

**10.** Procédé selon selon la revendication 2 caractérisé par le fait que l'alcanol répond à la formule générale (II) dans laquelle R' représente un radical carbocyclique saturé ou comprenant 1 ou 2 insaturations dans le cycle, ayant généralement de 3 à 7 atomes de carbone, de préférence, 6 atomes de carbone dans le cycle ; ledit cycle pouvant être éventuellement substitué.

**11.** Procédé selon l'une des revendications 2 et 8 caractérisé par le fait que l'alcanol répondant à la formule générale (II) est le méthanol, l'éthanol, l'isopropanol ou l'éthylène glycol.

**12.** Procédé selon l'une des revendications 1 à 11 caractérisé par le fait que le catalyseur de l'invention répond à la formule suivante :

$$MPO_4\ (Im)_p \qquad (III)$$

dans laquelle :

- M représente:
  - . une terre rare trivalente $M_3$,
  - . ou un mélange d'au moins une terre trivalente $M_3$ et d'au moins un élément choisi parmi les métaux alcalins $M_1$ et les métaux alcalino-terreux $M_2$ avec la relation :

$$M = \alpha M_1^{+} + \beta M_2^{++} + \gamma M_3^{3+} \text{ et } \alpha + 2\beta + 3\gamma = 3$$

- Im correspond à un composé d'imprégnation basique constitué de métal alcalino-terreux, de préférence de métal alcalin et leurs mélanges associés à un contre-anion pour assurer la neutralité électrique,
- $\alpha$ est un coefficient compris entre 0 et 3, avantageusement supérieur à 0,01 et au plus égal à 0,5, de préférence compris entre 0,05 et 0,2,
- $\beta$ est un coefficient compris entre 0 et 3/2, de préférence entre 0 et 1/3 ou bien 1 $\pm$ 0,1,
- $\gamma$ est un coefficient compris entre 0 et 1, avantageusement au moins égal à 1/3, de préférence à 1/2,
- p est inférieur à 0,5 et avantageusement compris entre 0,04 et 0,25.

**13.** Procédé selon la revendication 12 caractérisé par le fait que le catalyseur de l'invention répond à la formule (III) dans laquelle :

- $M_1$ est choisi dans le groupe des éléments de la colonne 1A et leurs mélanges, de préférence les métaux alcalins tels que le lithium, le sodium, le potassium, le rubidium et le césium,
- $M_2$ est choisi dans le groupe des éléments de la colonne 2A et leurs mélanges, de préférence les métaux alcalino-terreux tels que le béryllium, le magnésium, le calcium, le strontium et le baryum,
- $M_3$ est choisi dans le groupe des terres rares trivalentes choisies parmi les lanthanides, l'yttrium le scandium et leurs mélanges, de préférence les lanthanides tels que le lanthane, le cérium, le praséodyme, le néodyme, le samarium, l'europium, le gadolinium, le terbium, le dysprosium, l'holmium, l'erbium, le thulium, l'ytterbium et le lutécium.

**14.** Procédé selon l'une des revendications 1 à 13 caractérisé par le fait que le catalyseur de l'invention est un orthophosphate de métal de terre rare trivalente, de préférence de terre rare cérique, éventuellement dopé par un métal alcalin et/ou alcalino-terreux.

**15.** Procédé selon l'une des revendications 12 à 14 caractérisé par le fait que le catalyseur de l'invention répond à la formule (III) dans laquelle M est le lanthane, le cérium et le samarium dopé par un métal alcalin.

**16.** Procédé selon l'une des revendications 12 à 15 caractérisé par le fait que le catalyseur de l'invention a une teneur en agent dopant telle que le pourcentage pondéral de l'agent dopant par rapport au phosphate de terre trivalente est compris entre 0 et 25 %, de préférence entre 3 et 15%.

**17.** Procédé selon l'une des revendications 12 à 16 caractérisé par le fait que le catalyseur de l'invention répondant à la formule (III) dans laquelle $\alpha$ et $\beta$ sont égaux à 0, est calciné, à haute température avant sa mise en oeuvre.

**18.** Procédé selon la revendication 17 caractérisé par le fait que la calcination a lieu à une température de calcination est comprise entre 500°C à 1000°C, de préférence entre 700°C et 800°C, pendant une durée variant de 1 à 15 heures, de préférence de 3 à 6 heures.

**19.** Procédé selon l'une des revendications 1 à 18 caractérisé par le fait que catalyseur de l'invention répond à la formule (III) dans laquelle M est le lanthane, le cérium et le samarium dopé par du césium.

20. Procédé selon l'une des revendications 1 à 19 caractérisé par le fait que l'alcanol est mis en oeuvre en une quantité telle que le rapport entre le nombre de fonctions hydroxyle apportées par l'alcanol et le nombre de fonctions hydroxyle du composé phénolique de formule (I) varie entre 0,5 et 500, de préférence entre 1 et 5.

21. Procédé selon l'une des revendications 1 à 20 caractérisé par le fait la productivité pondérale et horaire du catalyseur est comprise entre 0,1 et 20 $h^{-1}$, de préférence entre 1 et 5 $h^{-1}$.

22. Procédé selon l'une des revendications 1 à 21 caractérisé par le fait que le rapport volumique entre le gaz vecteur et le composé phénolique varie entre 0 et 10, de préférence entre 0,1 et 2,0.

23. Procédé selon l'une des revendications 1 à 22 caractérisé par le fait que la température de la réaction de O-alkylation est généralement comprise entre 150°C et 500°C, et, de préférence de 200°C à 350°C.

24. Procédé selon l'une des revendications 1 à 23 caractérisé par le fait que le temps de contact est compris entre 0,5 et 100 secondes, de préférence entre 1 et 10 secondes.

25. Procédé selon l'une des revendications 1 à 24 caractérisé par le fait que le rapport molaire entre l'eau et le composé phénolique peut varier entre 0 et 5, et préférence entre 0,1 et 1.

26. Procédé selon l'une des revendications 1 à 25 caractérisé par le fait que l'on vaporise chacun des réactifs, composé phénolique, alcanol et éventuellement eau et que l'on met ensuite les réactifs en contact avec le catalyseur.

27. Procédé selon les revendications 1, 12 à 26 caractérisé par le fait que l'on prépare du gaïacol éventuellement associé au vératrole et du guétol par réaction de la pyrocatéchine respectivement avec du méthanol et de l'éthanol : la réaction ayant lieu en phase vapeur, en présence d'un orthophosphate de lanthane, de cérium ou de samarium éventuellement dopé par du césium.

28. Procédé selon les revendications 1, 12 à 26 caractérisé par le fait que l'on prépare l'anisole par réaction du phénol et du méthanol le p-méthoxyphénol éventuellement associé au p-diméthoxybenzène par réaction de l'hydroquinone et du méthanol ; l'éthylène dioxybenzène par réaction de la pyrocatéchine et de l'éthylène glycol : la réaction ayant lieu en phase vapeur, en présence d'un orthophosphate de lanthane, de cérium ou de samarium éventuellement dopé par du césium.

## Patentansprüche

1. Verfahren zur O-Alkylierung einer Phenolverbindung, dadurch gekennzeichnet, daß man die Phenolverbindung in der Gasphase in Gegenwart einer wirksamen Menge eines Katalysators, der aus Orthophosphaten dreiwertiger Seltenen Erdmetallen ausgewählt ist, mit einem Alkanol reagieren läßt.

2. Verfahren nach Anspruch 1, dadurch gekennzeichnet, daß die Phenolverbindung der allgemeinen Formel (I)

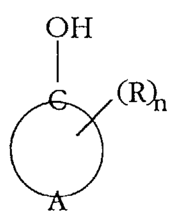

(I)

entspricht, in der:

- A einen monocyclischen oder polycyclischen, aromatischen carbocyclischen Rest oder einen zweiwertigen Rest darstellt, der aus einer Verkettung zweier oder mehrerer monocyclischer, aromatischer carbocyclischer Reste besteht,
- R einen oder mehrere Substituenten, identisch oder verschieden, darstellt,
- n eine ganze Zahl von weniger oder gleich 5 ist,

und daß der Alkohol der allgemeinen Formel (II)

$$R'\text{-}OH \quad (II)$$

entspricht, in der:

- R' ein Kohlenwasserstoffrest mit 1 bis 24 Kohlenstoffatomen darstellt, der ein linearer oder verzweigter, gesättigter oder ungesättigter, acyclischer aliphatischer Rest; ein monocyclischer oder polycyclischer, gesättigter oder ungesättigter cycloaliphatischer Rest; ein linearer oder verzweigter, gesättigter oder ungesättigter aliphatischer Rest, der einen cyclischen Substituenten enthält, sein kann.

**3.** Verfahren nach Anspruch 1 oder 2, dadurch gekennzeichnet, daß die Phenolverbindung der Formel (I) entspricht, in der der oder die Reste R eine der folgenden Gruppen darstellen:

- ein Wasserstoffatom,
- einen linearen oder verzweigten Alkylrest mit 1 bis 6 Kohlenstoffatomen, vorzugsweise mit 1 bis 4 Kohlenstoffatomen,
- einen linearen oder verzweigten Alkenylrest mit 2 bis 6 Kohlenstoffatomen, vorzugsweise mit 2 bis 4 Kohlenstoffatomen,
- einen linearen oder verzweigten Alkoxyrest mit 1 bis 6 Kohlenstoffatomen, vorzugsweise mit 1 bis 4 Kohlenstoffatomen,
- einen Acylrest mit 2 bis 6 Kohlenstoffatomen,
- einen Rest der Formel:

• $R_1$-OH

• $R_1$-$COOR_2$

• $R_1$-CHO

• $R_1$-$NO_2$

• $R_1$-CN

• $R_1$-$(NR_2)_2$

• $R_1$-CO-$(NR_2)_2$

• $R_1$-X

• $R_1$-$CF_3$

wobei in diesen Formeln $R_1$ eine Valenzbindung oder einen gesättigten oder ungesättigten, linearen oder verzweigten, zweiwertigen Kohlenwasserstoffrest mit 1 bis 6 Kohlenstoffatomen darstellt; $R_2$ ein Wasserstoffatom oder einen linearen oder verzweigten Alkylrest mit 1 bis 6 Kohlenstoffatomen darstellt; X ein Halogenatom, vorzugsweise ein Chlor-, Brom- oder Fluoratom, darstellt.

**4.** Verfahren nach einem der Ansprüche 1 bis 3, dadurch gekennzeichnet, daß die Phenolverbindung der Formel (I) entspricht, in der:

- R darstellt:

  - ein Wasserstoffatom,
  - eine OH-Gruppe,
  - einen linearen oder verzweigten Alkylrest mit 1 bis 6 Kohlenstoffatomen,
  - einen linearen oder verzweigten Alkenylrest mit 2 bis 6 Kohlenstoffatomen,
  - einen linearen oder verzweigten Alkoxyrest mit 1 bis 6 Kohlenstoffatomen,
  - eine CHO-Gruppe,
  - eine Acylgruppe mit 2 bis 6 Kohlenstoffatomen,
  - eine $COOR_2$-Gruppe, wobei $R_2$ die zuvor angegebene Bedeutung hat, • eine $NO_2$-Gruppe,
  - ein Halogenatom, vorzugsweise Fluor, Chlor und Brom,
  - eine $CF_3$-Gruppe;

- n eine ganze Zahl 0, 1, 2 oder 3 ist.

**5.** Verfahren nach einem der Ansprüche 1 bis 4, dadurch gekennzeichnet, daß die Phenolverbindung der Formel (I) entspricht, in der der Rest A darstellt:

- einen monocyclischen oder polycyclischen, aromatischen carbocyclischen Rest mit Ringen, die untereinander ein orthokondensiertes System bilden können, das der Formel (Ia)

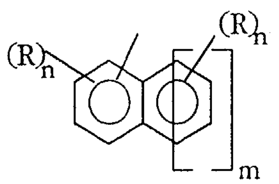

(Ia)

  entspricht, wobei in der Formel (Ia) m eine ganze Zahl von 0, 1 oder 2 darstellt und die Symbole R und n, jeweils identisch oder verschieden, die zuvor angegebene Bedeutung haben;
- einen Rest, der aus einer Verkettung von zwei oder mehreren monocyclischen, aromatischen carbocyclischen Resten besteht, die der Formel (Ib)

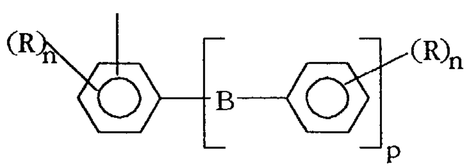

(Ib)

  entsprechen, wobei in der Formel (Ib) die Symbole R und n, jeweils identisch oder verschieden, die zuvor angegebene Bedeutung haben, p eine ganze Zahl von 0, 1, 2 oder 3 ist und B darstellt:
- eine Valenzbindung;
- einen Alkylen- oder Alkylidenrest mit 1 bis 4 Kohlenstoffatomen, vorzugsweise einen Methylen- oder Isopropylidenrest;
- eine der folgenden Gruppen:
  -O-, -CO-, -COO-, -OCOO-, -S-, -SO-, $SO_2$-,

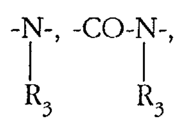

  wobei in diesen Formeln $R_3$ ein Wasserstoffatom oder einen Alkylrest mit 1 bis 4 Kohlenstoffatomen, einen

Cyclohexylrest oder einen Phenylrest darstellt.

**6.** Verfahren nach Anspruch 5, dadurch gekennzeichnet, daß die Phenolverbindung der Formel (I) einen Rest A darstellt, der den Formeln (Ia) und (Ib) entspricht, in denen:

- R ein Wasserstoffatom, eine Hydroxylgruppe, eine CHO-Gruppe, eine $NO_2$-Gruppe, einen linearen oder verzweigten Alkyl- oder Alkoxyrest mit 1 bis 6 Kohlenstoffatomen, vorzugsweise mit 1 bis 4 Kohlenstoffatomen, darstellt;
- B eine Valenzbindung, einen Alkylen- oder Alkylidenrest mit 1 bis 4 Kohlenstoffatomen oder ein Sauerstoffatom bedeutet;
- m gleich 0 oder 1 ist;
- n gleich 0, 1 oder 2 ist;
- p gleich 0 oder 1 ist.

**7.** Verfahren nach einem der Ansprüche 1 bis 6, dadurch gekennzeichnet, daß die Phenolverbindung der Formel (I) ausgewählt ist aus:

- Phenol
- Hydrochinon
- Pyrocatechin
- 2-Methoxyphenol
- 3-Methoxyphenol
- 4-Methoxyphenol
- 2-Ethoxyphenol
- 3-Ethoxyphenol
- 4-Ethoxyphenol
- Salicylaldehyd
- p-Hydroxybenzaldehyd
- Methylsalicylat
- p-Hydroxybenzoesäuremethylester
- 2-Chlorphenol
- 4-Chlorphenol
- 2-Nitrophenol
- 4-Nitrophenol
- N-Acetyl-p-Aminophenol
- Vanillin
- Isovanillin
- 2-Hydroxy-5-acetamidobenzaldehyd
- 2-Hydroxy-5-Propionamidobenzaldehyd
- 4-Allyloxybenzaldehyd
- 2,4-Dichlorphenol
- 2,6-Dichlorphenol
- Methylhydrochinon
- Pyrogallol
- 4-Bromvanillin
- 4-Hydroxyvanillin
- 2,4,6-Trichlorphenol
- 3,5-Dimethoxy-4-hydroxybenzaldehyd
- 6-Brom-2-naphtol.

**8.** Verfahren nach Anspruch 2, dadurch gekennzeichnet, daß das Alkanol der allgemeinen Formel (II) entspricht, in der R' einen linearen oder verzweigten Alkyl-, Alkenyl-, Alkadienyl- oder Alkinylrest mit vorzugsweise 1 bis 24 Kohlenstoffatomen darstellt, wobei die Kohlenwasserstoffkette gegebenenfalls:

- unterbrochen sein kann durch eine der folgenden Gruppen:
  -O-, -CO-, -COO-, -OCOO-, -S-, $-SO_2$,

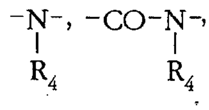

wobei in diesen Formel $R_4$ Wasserstoff oder einen linearen oder verzweigten Alkylrest mit 1 bis 4 Kohlenstoffatomen, vorzugsweise einen Methyl- oder Ethylrest, darstellt; und/oder

- einen der folgenden Substituenten enthalten kann:

  -OH, -OCOO-, $-COOR_4$, -CHO, $-NO_2$, -X, $-CF_3$ wobei in diesen Formeln $R_4$ die zuvor angegebene Bedeutung hat.

**9.** Verfahren nach Anspruch 2, dadurch gekennzeichnet, daß das Alkanol der allgemeinen Formel (II) entspricht, in der R' einen linearen oder verzweigten, gesättigten oder ungesättigten, acyclischen aliphatischen Rest darstellt, der einen gegebenenfalls substituierten ringförmigen Substituenten enthält, wobei der Rest mit dem Ring über eine Valenzbindung oder über eine der folgenden Gruppen verbunden sein kann:

-O-, -CO-, -COO-, -OCOO-, -S-, $-SO_2$,

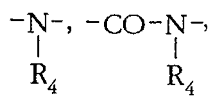

wobei in diesen Formeln $R_4$ die zuvor in Anspruch 8 angegebene Bedeutung hat.

**10.** Verfahren nach Anspruch 2, dadurch gekennzeichnet, daß das Alkanol der allgemeinen Formel (II) entspricht, in der R' einen gesättigten carbocyclischen Rest oder einen carbocyclischen Rest mit 1 oder 2 ungesättigten Bindungen im Ring darstellt, der im allgemeinen 3 bis 7 Kohlenstoffatome, vorzugsweise 6 Kohlenstoffatome, in dem Ring enthält, wobei dieser Ring gegebenenfalls substituiert sein kann.

**11.** Verfahren nach Anspruch 2 oder 8, dadurch gekennzeichnet, daß das Alkanol der allgemeinen Formel (II) Methanol, Ethanol, Isopropanol oder Ethylenglykol ist.

**12.** Verfahren nach einem der Ansprüche 1 bis 11, dadurch gekennzeichnet, daß der erfindungsgemäße Katalysator der folgenden Formel

$$MPO_4(Im)_p \qquad \text{(III)}$$

entspricht, in der:

- M darstellt:

  - eine dreiwertige Seltene Erde $M_3$,
  - oder eine Mischung mindestens einer dreiwertigen Seltenen Erde $M_3$ und mindestens eines Elements, das ausgewählt ist aus Alkalimetallen $M_1$ und Erdalkalimetallen $M_2$ im folgenden Verhältnis:

$$M = \alpha M_1^{+} + \beta M_2^{++} + \gamma M_3^{3+} \text{ und } \alpha + 2\beta + 3\gamma = 3;$$

  - Im einer basischen Imprägnierverbindung entspricht, die aus einem Erdalkalimetall und vorzugsweise einem Alkalimetall und deren Mischungen besteht, die mit einem Gegenion verbunden sind, um die elektrische Neutralität zu gewährleisten;
  - $\alpha$ ein Koeffizient zwischen 0 und 3, vorzugsweise größer als 0,01 und höchstens gleich 0,5, insbesondere zwischen 0,05 und 0,2, ist;
  - $\beta$ ein Koeffizient zwischen 0 und 3/2, vorzugsweise zwischen 0 und 1/3 oder auch 1 ± 0,1, ist;
  - $\gamma$ ein Koeffizient zwischen 0 und 1, vorzugsweise von mindestens gleich 1/3, insbesondere von 1/2, ist;

- p kleiner als 0,5, vorzugsweise zwischen 0,04 und 0,25, ist.

**13.** Verfahren nach Anspruch 12, dadurch gekennzeichnet, daß der erfindungsgemäße Katalysator der Formel (III) entspricht, in der:

- $M_1$ ausgewählt ist aus der Gruppe von Elementen der Gruppe 1A und ihren Mischungen, vorzugsweise aus Alkalimetallen wie Lithium, Natrium, Kalium, Rubidium und Cäsium;
- $M_2$ ausgewählt ist aus der Gruppe von Elementen der Gruppe 2A und ihren Mischungen, vorzugsweise Erdalkalimetallen wie Beryllium, Magnesium, Calcium, Strontium und Barium;
- $M_3$ ausgewählt ist aus der Gruppe von dreiwertigen Seltenen Erden, die ausgewählt sind aus Lanthaniden, Yttrium, Scandium und ihren Mischungen, vorzugsweise Lanthaniden wie Lanthan, Cer, Praseodym, Neodym, Samarium, Europium, Gadolinium, Terbium, Dysprosium, Holmium, Erbium, Thulium, Ytterbium und Lutetium.

**14.** Verfahren nach einem der Ansprüche 1 bis 13, dadurch gekennzeichnet, daß der erfindungsgemäße Katalysator ein Orthophosphat einer dreiwertigen Seltenen Erde, vorzugsweise der Seltenen Erde Cer, ist, die gegebenenfalls mit einem Alkalimetall und/oder Erdalkalimetall dotiert ist.

**15.** Verfahren nach einem der Ansprüche 12 bis 14, dadurch gekennzeichnet, daß der erfindungsgemäße Katalysator der Formel (III) entspricht, in der M Lanthan, Cer und Samarium ist, das mit einem Alkalimetall dotiert ist.

**16.** Verfahren nach einem der Ansprüche 12 bis 15, dadurch gekennzeichnet, daß der erfindungsgemäße Katalysator einen Gehalt an Dotiermittel, berechnet als Gewichtsprozent des Dotierreagenzes in bezug auf das Phosphat der dreiwertigen Seltenen Erde, zwischen 0 und 25 %, vorzugsweise zwischen 3 und 15 %, aufweist.

**17.** Verfahren nach einem der Ansprüche 12 bis 16, dadurch gekennzeichnet, daß der erfindungsgemäße Katalysator, der der Formel (III) entspricht, in der $\alpha$ und $\beta$ gleich 0 sind, vor seiner Verwendung bei hoher Temperatur calciniert wird.

**18.** Verfahren nach Anspruch 17, dadurch gekennzeichnet, daß die Calcinierung bei einer Calcinierungstemperatur zwischen 500 °C und 1000 °C, vorzugsweise zwischen 700 °C und 800 °C, für eine Dauer von 1 bis 15 Stunden, vorzugsweise von 3 bis 6 Stunden, durchgeführt wird.

**19.** Verfahren nach einem der Ansprüche 1 bis 18, dadurch gekennzeichnet, daß der erfindungsgemäße Katalysator der Formel (III) entspricht, in der M Lanthan, Cer und Samarium ist, dotiert mit Cäsium.

**20.** Verfahren nach einem der Ansprüche 1 bis 19, dadurch gekennzeichnet, daß das Alkanol in einer solchen Menge eingesetzt wird, daß das Verhältnis zwischen der Zahl der Hydroxylfunktionen, die durch das Alkanol eingebracht werden, und der Zahl an Hydroxylfunktionen der Phenolverbindung der Formel (I) zwischen 0,5 und 500, vorzugsweise zwischen 1 und 5, variiert.

**21.** Verfahren nach einem der Ansprüche 1 bis 20, dadurch gekennzeichnet, daß die Produktivität des Katalysators, bezogen auf Gewicht und Stunde, zwischen 0,1 und 20 $h^{-1}$, vorzugsweise zwischen 1 und 5 $h^{-1}$, liegt.

**22.** Verfahren nach einem der Ansprüche 1 bis 21, dadurch gekennzeichnet, daß das Volumenverhältnis zwischen dem Schleppgas und der Phenolverbindung zwischen 0 und 10, vorzugsweise zwischen 0,1 und 2,0, variiert.

**23.** Verfahren nach einem der Ansprüche 1 bis 22, dadurch gekennzeichnet, daß die Reaktionstemperatur der O-Alkylierung im allgemeinen zwischen 150 °C und 500 °C, vorzugsweise zwischen 200 °C und 350 °C, liegt.

**24.** verfahren nach einem der Ansprüche 1 bis 23, dadurch gekennzeichnet, daß die Kontaktzeit zwischen 0,5 und 100 Sekunden, vorzugsweise zwischen 1 und 10 Sekunden, liegt.

**25.** Verfahren nach einem der Ansprüche 1 bis 24, dadurch gekennzeichnet, daß das Molverhältnis zwischen dem Wasser und der Phenolverbindung zwischen 0 und 5, vorzugsweise zwischen 0,1 und 1, variieren kann.

**26.** Verfahren nach einem der Ansprüche 1 bis 25, dadurch gekennzeichnet, daß man jedes der Reagenzien, die Phenolverbindung, das Alkanol und gegebenenfalls Wasser, verdampft und daß man dann die Reagenzien in Kontakt mit dem Katalysator bringt.

**27.** Verfahren nach den Ansprüchen 1 und 12 bis 26, dadurch gekennzeichnet, daß man Guajakol, gegebenenfalls zusammen mit Veratrol, und Guetol jeweils durch Reaktion des Pyrocatechins mit Methanol bzw. Ethanol herstellt, wobei die Reaktion in der Gasphase in Gegenwart eines Lanthan-, Cer- oder Samariumorthophosphats, das gegebenenfalls mit Cäsium dotiert ist, durchgeführt wird.

**28.** Verfahren nach den Ansprüchen 1 und 12 bis 26, dadurch gekennzeichnet, daß man Anisol durch Reaktion von Phenol und Methanol, p-Methoxyphenol, gegebenenfalls zusammen mit p-Dimethoxybenzol, durch Reaktion von Hydrochinon und Methanol; Ethylendioxybenzol durch Reaktion von Pyrocatechin und Ethylenglykol herstellt, wobei die Reaktion in der Gasphase in Gegenwart eines Lanthan-, Cer- oder Samarium-orthophosphats, das gegebenenfalls mit Cäsium dotiert ist, durchgeführt wird.

**Claims**

**1.** A process for O-alkylation of a phenolic compound, characterised by the fact that it consists in reacting said phenolic compound with an alkanol, in gaseous phase, in the presence of an effective amount of a catalyst selected from orthophosphates of trivalent rare earth metals.

**2.** A process according to Claim 1, characterised by the fact that the phenolic compound corresponds to the general formula (I):

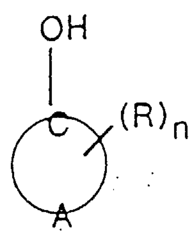

(I)

wherein:

- A symbolises a residue of a monocyclic or polycyclic aromatic carbocyclic radical or a divalent radical constituted by a chain of two or more monocyclic aromatic carbocyclic radicals,
- R represents one or more substitutes, which may be the same or different,
- n is a number less than or equal to 5.

with an alcohol which corresponds to the general formula (II):

$$R' - OH \qquad (II)$$

in said formula (II):

- R' represents a hydrocarbon radical with 1 to 24 carbon atoms which can be a linear or branched saturated or unsaturated acyclic aliphatic radical; a monocyclic or polycyclic saturated or unsaturated cycloaliphatic radical; a branched or linear saturated or unsaturated aliphatic radical which is a carrier of a cyclic substitute.

**3.** A process according to one of Claims 1 and 2, characterised by the fact that the phenolic compound corresponds to formula (I) in which the R radical(s) represent one of the following groups:

- a hydrogen atom,
- a branched or linear alkyl radical with 1 to 6 carbon atoms, preferably with 1 to 4 carbon atoms,
- a branched or linear alkenyl radical with 2 to 6 carbon atoms, preferably with 2 to 4 carbon atoms,
- a branched or linear alkoxy radical with 1 to 6 carbon atoms, preferably 1 to 4 carbon atoms,
- an acyl group with 2 to 6 carbon atoms,
- a radical of the formula:

$-R_1-OH$

$-R_1-COOR_2$

$-R_1-CHO$

$-R_1-NO_2$

$-R_1-CN$

$-R_1-(NR_2)_2$

$-R_1-CO-(NR_2)_2$

$-R_1-X$

$-R_1-CF_3$

in which formulae, $R_1$ represents a valency bond or a saturated or unsaturated, branched or linear, divalent hydrocarbon radical with 1 to 6 carbon atoms; $R_2$ represents a hydrogen atom or a branched or linear alkyl radical with 1 to 6 carbon atoms; X symbolises a halogen atom, preferably a chlorine, bromine or fluorine atom.

**4.** A process according to one of Claims 1 to 3, characterised by the fact that the phenolic compound corresponds to formula (I) in which:

- R represents:
  . a hydrogen atom
  . a OH group,
  . a branched or linear alkyl radical with 1 to 6 carbon atoms,
  . a branched or linear alkenyl radical with 2 to 6 carbon atoms,
  . a branched or linear alkoxy radical with 1 to 6 carbon atoms,
  . a -CHO group,
  . an acyl group with 2 to 6 carbon atoms,
  . a $-COOR_2$ group where $R_2$ has the meaning given hereinabove,
  . a $-NO_2$ group,
  . a halogen atom, preferably a fluorine, chlorine or bromine atom,
  . a $-CF_3$ group.
- n is a number equal to 0, 1, 2 or 3.

**5.** A process according to one of Claims 1 to 4, characterised by the fact that the phenolic compound corresponds to formula (I) in which the residue A represents:

- a monocyclic or polycyclic aromatic carbocyclic radical with cycles which can between them form an orthocondensed system corresponding to formula (Ia):

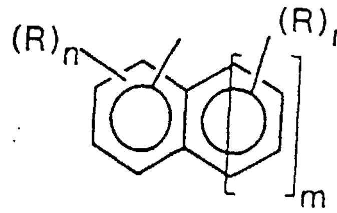

(Ia)

in which formula (1a) m represents a number equal to 0, 1 or 2 and the symbols R and n which may be the same or different have the meaning given hereinabove,

- a radical constituted by a chain of two or more monocyclic aromatic carbocyclic radicals corresponding to the formula (1b):

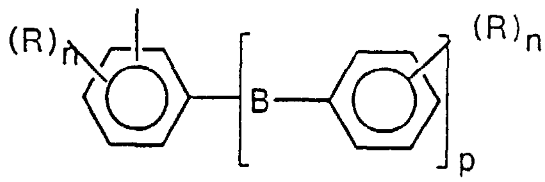

(Ib)

in which formula (1b), the symbols R and n which may be identical or different have the meaning given hereinabove, p is a number equal to 0, 1, 2 or 3 and B represents:

- a valency bond
- an alkylene or alkylidene radical with 1 to 4 carbon atoms, preferably a methylene or isopropylidene radical,
- one of the following groups:
  -O-, -CO-, -COO-, -OCOO-,
  -S-, -SO-, $-SO_2-$,

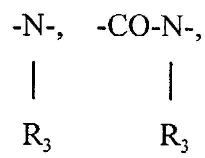

in these formulae, $R_3$ represents a hydrogen atom or an alkyl radical with 1 to 4 carbon atoms, a cyclohexyl or phenyl radical.

**6.** A process according to Claim 5, characterised by the fact that the phenolic compound of formula (I) has a residue A which corresponds to formulae (1a) and (1b) wherein:

- R represents a hydrogen atom, a hydroxyl group, a _CHO group, a $-NO_2$ group, a linear or branched alkyl or alkoxy radical with 1 to 6 carbon atoms, preferably with 1 to 4 carbon atoms,
- B symbolises a valency bond, an alkylidene or alkylene radical with 1 to 4 carbon atoms or an oxygen atom,
- m is equal to 0 or 1,
- n is equal to 0, 1 or 2,
- p is equal to 0 or 1.

**7.** A process according to one of Claims 1 to 6, characterised by the fact that the phenolic compound of formula (I) is selected from:

- phenol
- hydroquinone
- pyrocatechin
- 2-methoxy phenol
- 3-methoxy phenol

- 4-methoxy phenol
- 2-ethoxy phenol
- 3-ethoxy phenol
- 4-ethoxy phenol
- salicylic aldehyde
- p-hydroxybenzaldehyde
- methyl salicylate
- methylic ester of p-hydroxybenzoic acid
- 2-chloro phenol
- 4-chloro phenol
- 2-nitro phenol
- 4-nitro phenol
- N-acetyl p-aminophenol
- vanillin
- isovanillin
- 2-hydroxy 5-acetamidobenzaldehyde
- 2-hydroxy 5-propionamidobenzaldehyde
- 4-allyloxybenzaldehyde
- 2,4-dichlorophenol
- 2,6-dichlorophenol
- methylhydroquinone
- pyrogallol
- 4-bromovanillin
- 4-hydroxyvanillin
- 2,4,6-trichlorophenol
- 3,5-dimethoxy 4-hydroxybenzaldehyde
- 6-bromo 2-naphthol

**8.** A process according to Claim 2, characterised by the fact that the alkanol corresponds to the general formula (II) in which R' represents a linear or branched alkynyl, alkadienyl, alkenyl, alkyl radical with preferably 1 to 24 carbon atoms; the hydrocarbon chain possibly being able to be:

- interrupted by one of the following groups:
  -O-, -CO-, -COO-, OCOO-, -S-, $-SO_2-$,

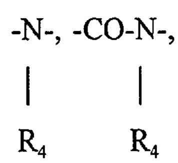

  in which formulae $R_4$ represents hydrogen or a linear or branched alkyl radical with 1 to 4 carbon atoms, preferably a methyl or ethyl radical,
- and/or being the carrier of one of the following substitutes:

$$-OH, -OCOO-, -COOR_4, -CHO, -NO_2, -X, -CF_3$$

  in which formulae $R_4$ has the meaning given hereinabove.

**9.** A process according to Claim 2, characterised by the fact that the alkanol corresponds to general formula (II) in which R' represents a linear or branched, saturated or unsaturated acyclic aliphatic residue which is the carrier of a cyclic substitute which may possibly be substituted; said cycle being able to be connected to the cycle by a valency bond or by one of the following groups:
-O-, -CO-, -COO-, -OCOO-, -S-, $-SO_2-$,

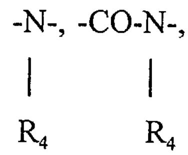

in which formulae $R_4$ has the meaning given hereinabove in Claim 8.

**10.** A process according to Claim 2, characterised by the fact that the alkanol corresponds to general formula (II) in which R' represents a saturated carbocyclic radical or comprising 1 or 2 insaturations in the cycle, usually with 3 to 7 carbon atoms, preferably with 6 carbon atoms in the cycle; said cycle possibly being able to be substituted.

**11.** A process according to one of Claims 2 and 8, characterised by the fact that the alkanol corresponding to general formula (II) is methanol, ethanol, isopropanol or ethylene glycol.

**12.** A process according to one of Claims 1 to 11, characterised by the fact that the catalyst of the invention corresponds to the following formula:

$$MPO_4\ (Im)_p \qquad (III)$$

in which:

- M represents:
  - . a $M_3$ trivalent rare earth,
  - . or a mixture of at least one $M_3$ trivalent earth and at least one element selected from the $M_1$ alkaline metals and the $M_2$ alkaline earth metals of equation:

$$M = \alpha M_1^{+} + \beta M_2^{++} + \gamma M_3^{3+} \text{ and } \alpha + 2\beta + 3\gamma = 3$$

- Im corresponds to a basic impregnation compound constituted by alkaline earth metals, preferably alkaline metal and their mixtures associated with a counter anion to provide electrical neutrality,
- $\alpha$ is a coefficient between 0 and 3, advantageously above 0.01 and at least equal to 0.5, preferably between 0.05 and 0.2,
- $\beta$ is a coefficient between 0 and 3/2, preferably between 0 and 1/3 or 1 $\pm$ 0.1,
- $\gamma$ is a coefficient between 0 and 1, advantageously at least equal to 1/3, preferably 1/2,
- p is less than 0.5 and advantageously between 0.04 and 0.25.

**13.** A process according to Claim 12, characterised by the fact that the catalyst of the invention corresponds to formula (III) in which:

- $M_1$ is selected from the group of elements in Column 1A and their mixtures, preferably alkaline metals such as lithium, sodium, potassium, rubidium and cesium,
- $M_2$ is selected from the group of elements in column 2A and their mixtures, preferably alkaline earth metals such as beryllium, magnesium, calcium, strontium and barium,
- $M_3$ is selected from the group of trivalent rare earths selected from lanthanides, yttrium, scandium and mixtures thereof, preferably lanthanides such as lanthanum, cerium, praseodymium, neodymium, samarium, europium, gadolinium, terbium, dysprosium, holmium, erbium, thulium, ytterbium and lutecium.

**14.** A process according to Claims 1 to 13, characterised by the fact that the catalyst of the invention is an orthophosphate of a trivalent rare earth metal, preferably a ceric rare earth, possibly doped with an alkaline metal and/or alkaline earth metal.

**15.** A process according to one of Claims 12 to 14, characterised by the fact that the catalyst of the invention corresponds to formula (III) in which M is lanthanum, cerium and samarium possibly doped with an alkaline metal.

**16.** A process according to one of Claims 12 to 15, characterised by the fact that the catalyst of the invention has a doping agent content such that the percentage by weight of the doping agent in relation to the phosphate of the trivalent earth is between 0 and 25%, preferably between 3 and 15%.

**17.** A process according to one of Claims 12 to 16, characterised by the fact that the catalyst of the invention which corresponds to formula (III) in which $\alpha$ and $\beta$ are equal to 0, is calcined at a high temperature prior to use.

**18.** A process according to Claim 17, characterised by the fact that the calcination operation takes place at a calcination temperature of between 500°C and 1000°C, preferably between 700°C and 800°C, for a period of between 1 and 15 hours, preferably of between 3 and 6 hours.

**19.** A process according to one of Claims 1 to 18, characterised by the fact that the catalyst of the invention corresponds to formula (III) in which M is lanthanum, cerium and samarium doped with cesium.

**20.** A process according to one of Claims 1 to 19, characterised by the fact that the amount of alkanol used is such that the ratio between the number of hydroxyl functions brought by the alkanol and the number of hydroxyl functions of the phenolic compound of formula (I) varies between 0.5 and 500, preferably between 1 and 5.

**21.** A process according to one of Claims 1 to 20, characterised by the fact that the productivity by weight and per hour of the catalyst is between 0.1 and 20 $h^{-1}$, preferably between 1 and 5 $h^{-1}$.

**22.** A process according to one of Claims 1 to 21, characterised by the fact that the ratio by volume between the vector gas and the phenolic compound varies between 0 and 10, preferably between 0.1 and 2.0.

**23.** A process according to one of Claims 1 to 22, characterised by the fact that the O-alkylation reaction temperature is usually between 150°C and 500°C, and, preferably between 200°C and 350°C.

**24.** A process according to one of Claims 1 to 23, characterised by the fact that the contact time is between 0.5 and 100 seconds, preferably between 1 and 10 seconds.

**25.** A process according to one of Claims 1 to 24, characterised by the fact that the molar ratio between the water and the phenolic compound can vary between 0 and 5, and preferably between 0.1 and 1.

**26.** A process according to one of Claims 1 to 25, characterised by the fact that each of the reagents, phenolic compound, alkanol and possibly water is vaporised and the reagents are then contacted with the catalyst.

**27.** A process according to Claims 1, 12 to 26, characterised by the fact that the guaiacol is prepared which may be associated with veratrol and guetol by reacting pyrocatechin respectively with methanol and ethanol: the reaction takes place in vapour phase, in the presence of a lanthanum, cerium or samarium orthophosphate, possibly doped with cesium.

**28.** A process according to Claims 1, 12 to 26, characterised by the fact that the anisole is prepared by reacting phenol and methanol; the p-methoxyphenol which may be associated with p-dimethoxybenzene by reacting hydroquinone and methanol; the ethylene dioxybenzene by reacting pyrocatechin and ethylene glycol: the reaction taking place in vapour phase, in the presence of a lanthanum, cerium or samarium orthophosphate possibly doped with cesium.